# EUROPEAN PATENT SPECIFICATION

(11) **EP 3 946 737 B1**
(45) Date of publication and mention of the grant of the patent: **04.06.2025**
(21) Application number: 20783112.4
(22) Date of filing: 02.04.2020
(51) Int. Cl.: B01L 3/00, G01N 9/10, G01N 21/64, G01N 21/93, G01N 23/12, G01N 33/543

(54) **METHOD FOR RE-USING HAPTEN-COATED PROBE IN AN IMMUNOASSAY**
VERFAHREN ZUR WIEDERVERWENDUNG EINER HAPTENBESCHICHTETEN SONDE IN EINEM IMMUNOASSAY
PROCÉDÉ DE RÉUTILISATION D'UNE SONDE REVÊTUE D'HAPTÈNE DANS UN IMMUNOESSAI

(30) Priority: 03.04.2019 US 201962828865 P; 24.12.2019 US 201962953340 P
(43) Date of publication of application: 09.02.2022
(73) Proprietor: Access Medical Systems, Ltd., Palo Alto, CA 94303 (US)
(72) Inventor: ZUK, Robert F., Palo Alto, California 94303 (US); XIA, Qing, Palo Alto, California 94303 (US); WU, Jenchieh, Palo Alto, California 94303 (US)
(74) Representative: Goodacre, Jonathan David
(86) International application number: PCT/US2020/026466
(87) International publication number: WO 2020/206175

(56) References cited:
- WO-A1-2016/183092
- WO-A1-2021/071903
- WO-A1-98/18968
- US-A- 5 108 896
- US-A1- 2006 276 972
- US-A1- 2017 153 231
- US-A1- 2018 180 606
- US-A1- 2018 180 606
- KIM H-O ET AL: "REUSABILITY OF AVIDIN-BIOTINYLATED IMMUNOGLOBULIN Y COLUMNS IN IMMUNOAFFINITY CHROMATOGRAPHY", ANALYTICAL BIOCHEMISTRY, ACADEMIC PRESS, AMSTERDAM, NL, vol. 268, no. 2, 1 March 1999 (1999-03-01), pages 383 - 397, XP002905439, ISSN: 0003-2697, DOI: 10.1006/ABIO.1998.3081

## Description

### FIELD OF THE INVENTION

The present invention related to an immunoassay method, which re-uses a hapten-immobilized test probe for quantitating an analyte in different samples, from about 3 to 10 times. The method uses a dual antibody conjugate solution in each cycle and regenerates the hapten-coated test probe by dipping the test probe in an acidic solution having pH about 1-4, after the completion of each cycle of reaction.

### BACKGROUND OF THE INVENTION

Cost containment is a major goal for healthcare providers worldwide. In vitro diagnostics (IVD) is no exception, where the clinical utility of biomarkers in the diagnosis and prognosis has become standard in-patient management. Immunoassay technology is large portion of the IVD industry and is steadily growing, about 3%/year in the U.S. and 15-20%/year in developing countries. In some cases, such as serial measurements for cardiac markers in diagnosing myocardial infarction, cost can limit the appropriate amount of testing.

Typical approaches to reducing the cost of immunoassays entail minimizing manufacturing expenses for materials, labor, and facilities overhead.

Any method to recycle immune reagents typically centers upon disassociating the immune complex with a denaturing agent such as an acidic/basic pH solution, organic solvents, chaotropic agents, etc. The denaturation step changes the antibody charge, hydration, hydrogen bonding and tertiary structure where it no longer binds to antigen. Exposing the antibody back to the initial binding conditions close to physiologic pH and ionic strength, is expected to restore original binding activity, however, few antibodies can tolerate repeated exposures to denaturation conditions without adversely impacting some aspect of their binding properties and consequently, assay performance.

There is a need for reducing the cost of immunoassays, while maintaining the clinical performance at the same time.

### SUMMARY OF THE INVENTION

In one aspect, the present invention provides a method of detecting an analyte in multiple liquid samples comprising an analyte, comprising the steps of:
(a) dipping a probe in an aqueous solution having pH of 6.0-8.5 to pre-read the fluorescent signal of the probe tip, wherein the probe comprises a hapten immobilized on the tip of the probe, and the diameter of the tip surface is ≤ 5 mm;
(b) forming an immunocomplex comprising the analyte from a sample, a capture antibody, and a signal antibody on the probe tip, wherein the capture antibody and the signal antibody are two different antibodies against the analyte, the capture antibody is covalently linked to an anti-hapten antibody against the hapten and the signal antibody is conjugated with fluorescent labels;
(c) dipping the probe tip into a washing vessel containing a wash solution;
(d) determining the analyte concentration in the sample by measuring the fluorescent signal of the immunocomplex at the probe tip, subtracting the pre-read fluorescent signal of (a), and quantitating the subtracted signal against a calibration curve;
(e) dipping the probe tip in an acidic solution having pH about 1.0-4.0 to elute the immunocomplex from the probe tip; and
(f) repeating the steps (a)-(e) except in step (b) with the analyte from a new sample, whereby the analyte in each of the multiple liquid samples is detected.

In another aspect, the present invention provides a method of detecting an analyte in multiple liquid samples, comprising the steps of:
(a) dipping a probe in an aqueous solution having pH of 6.0-8.5 to pre-read the fluorescent signal of the probe tip, wherein the probe comprises a first hapten immobilized on the tip of the probe, and the diameter of the tip surface is ≤ 5 mm;
(b) forming a first immunocomplex comprising the analyte from a sample, a capture antibody, and a signal antibody on the probe tip, wherein the capture antibody and the signal antibody are two different antibodies against the analyte, the capture antibody is covalently linked to an anti-first hapten antibody against the first hapten and the signal antibody is conjugated with a second hapten, the first hapten and the second hapten are different;
(c) dipping the probe tip in a wash solution;
(d) dipping the probe tip in an amplification solution comprising an anti-second hapten antibody or streptavidin conjugated to fluorescent labels to form a second immunocomplex comprising the analyte, the capture antibody, the signal antibody, the second hapten, and the anti-second hapten antibody or streptavidin on the probe tip;
(e) determining the analyte concentration in the sample by measuring the fluorescent signal of the second immunocomplex at the probe tip, subtracting the pre-read fluorescent signal of (a), and quantitating the subtracted signal against a calibration curve;
(f) dipping the probe tip in an acidic solution having pH about 1.0-4.0 to elute the immunocomplexes from the probe tip; and
(g) repeating the same steps (a)-(f) except in step (b) with the analyte from a new sample, whereby the analyte in each of the multiple liquid samples is detected.

In another aspect, the present invention provides a method of detecting an analyte in multiple liquid samples, comprising the steps of:
(a) dipping a probe in an aqueous solution having pH of 6.0-8.5 to pre-read the fluorescent signal of the probe tip, wherein the probe comprises a hapten immobilized on the tip of the probe, and the diameter of the tip surface is ≤ 5 mm;
(b) forming an immunocomplex comprising the analyte from a sample, a capture antibody, and a signal antibody on the probe tip, wherein the capture antibody and the signal antibody are two different antibodies against the analyte, the capture antibody is covalently linked to an anti-hapten antibody against the hapten and the signal antibody is conjugated with fluorescent labels;
(c) dipping the probe tip into a washing vessel containing a wash solution;
(d) reading a first fluorescent signal of the immunocomplex at the probe tip;
(e) dipping the probe tip in an acidic solution having pH about 1.0-4.0 and containing guanidium chloride;
(f) reading a second fluorescent signal of the acid-treated probe tip,
(g) subtracting the second fluorescent signal from the first fluorescent signal, and quantitating the subtracted signal against a calibration curve;
(h) dipping the probe tip in a chaotropic agent different from guanidium chloride; and
(i) repeating the same steps (b)-(h) except in step (b) with a new sample solution comprising a new sample, whereby the analyte in each of the multiple liquid samples is detected.

### BRIEF DESCRIPTION OF THE DRAWINGS

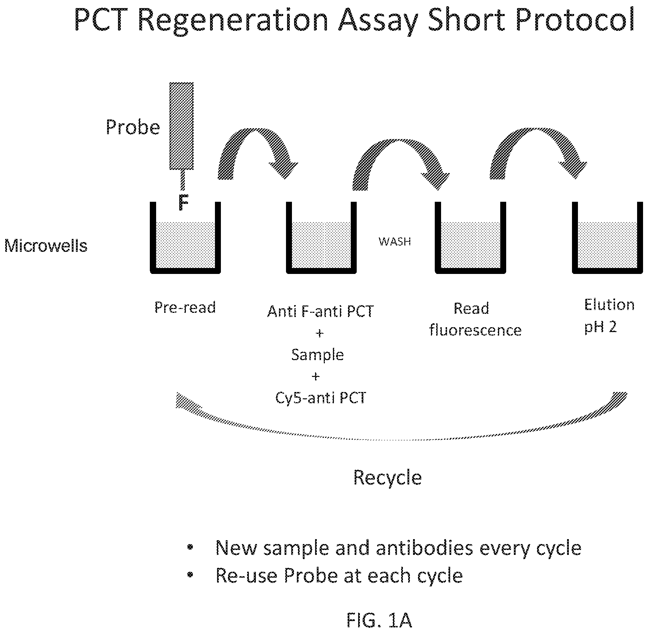

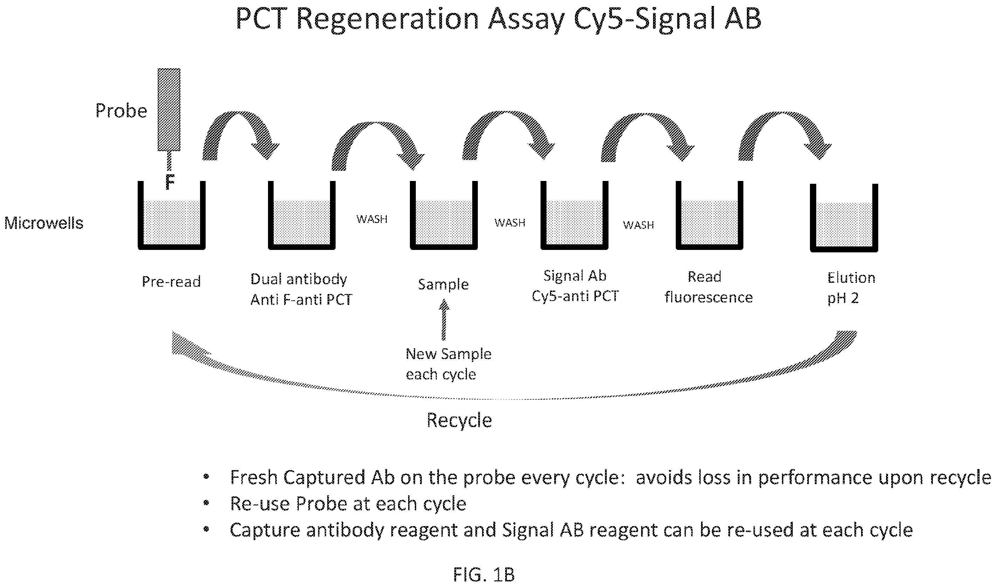

FIGs. 1A and 1B depict a procalcitonin (PCT) assay configuration with a fluorescein (F) coated probe, a conjugate of anti F/anti-PCT as capture antibody (AB), Cy5-anti PCT as signal AB, and pH 2 elution. The initial step in the recycle protocol is the "pre-read" which measures the intrinsic fluorescence of the glass probe and any residual fluorescence retained on the probe tip from the previous cycle. This signal is subtracted from the final Read step at the end of the assay to derive the immuno-specific fluorescence signal. The pH 2 elution step strips the immune complex from the probe surface including the capture AB. Every cycle has a fresh coating of capture AB, which is critical in maintaining assay performance throughout multiple cycles. The 1 mm diameter probe has such small surface area that any binding by the capture AB or signal AB consumes only a negligible amount of those reagents, consequently allowing their re-use in multiple cycles. The fluorescein coated probe can tolerate the multiple cycles through the pH 2 elution and back to neutral pH, enabling its re-use.
FIG. 2 shows data of PCT assays following protocol A, v1 with the fluorescein probe format described in FIG. 1B.
FIG. 3 shows the data of PCT standards in the fluorescein coated probe format (FIG. 1B) with protocol A, v1.
FIG. 4 shows that the results with PCT clinical serum samples having correlation (R² = 0.95) of the recycle assay with a commercial IVD instrument (Biomerieux *Vidas).*
FIG. 5 presents results applying the recycle assay (Protocol A, v2) to another immunoassay, cardiac troponin I (TnI). The format is as FIG. 1B, except capture AB was anti F linked to anti-TnI and signal AB was Cy5 -anti-TnI.
FIG. 6 illustrates an alternative recycle assay. The format is identical to FIG. 1B, except the signal AB is labeled with a different fluorescent dye, Alexa Fluor 647.
FIG. 7 contains data of different PCT samples up 10 cycles using the Alexa Fluor 647 labeled signal AB (Protocol A, v3).
FIG. 8 shows the recycle assay using a different hapten/anti hapten format. The format is similar to FIG. 1B, except that the probe is coated with digoxiginnen and the capture AB is an anti digoxiginnen-anti-PCT conjugate.
FIG. 9 presents PCT levels up to 10 cycles using the digoxiginnen format (Protocol A, v4). Results are very similar to the fluorescein probe format in FIG. 3.
FIG. 10 shows a recycle format with an additional amplification reagent. In this example, the signal antibody is labeled with biotin and there is a Cy5-anti-Biotin-FICOLL^{®} reagent. FICOLL^{®} (copolymers of sucrose and epichlorohydrin) is several million Daltons in molecular weight and serves to carry multiple dye labeled anti biotin antibodies. The amplification reagent would bind to biotin containing immune complexes on the surface of the probe and would yield greater signal than the direct dye labeling of the signal AB.
FIG. 11 shows PCT assays performed with the recycle format in FIG. 10 with Protocol B, v1 using pH 2 elution.
FIG. 12 illustrates an alternative amplification reagent. The recycle format is similar to FIG. 10, except the amplification reagent is Cy5-streptavidin-FICOLL^{®}. Streptavidin is smaller in molecular size than an antibody, therefore more streptavidin molecules can be linked to the FICOLL^{®} polymer to generate more fluorescence signal compared to anti-biotin linked FICOLL^{®}. Streptavidin-based amplification reagents offer a further advantage in that it has extremely high binding affinity to biotin.
FIG. 13 shows the PCT results with the streptavidin amplification reagent using FIG. 12, Protocol B, v2 with pH 2 elution.
FIG. 14 shows the recycle format similar to FIG. 12, except after the pH 2 step, the probe is further exposed to DMSO (100%) for a second elution.
FIG. 15 contains the results of the recycle assay of 3 PCT levels with the dual pH 2 and DMSO elution as in FIG. 14 using Protocol C.

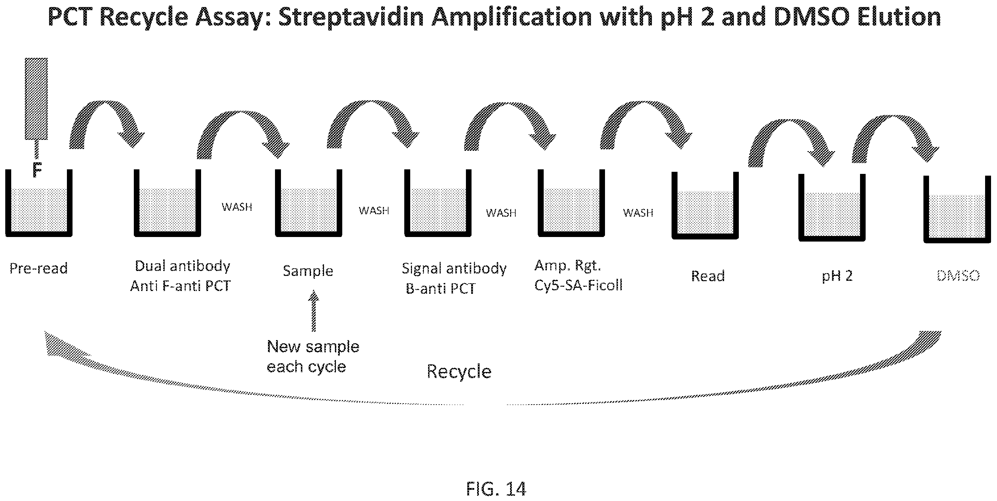

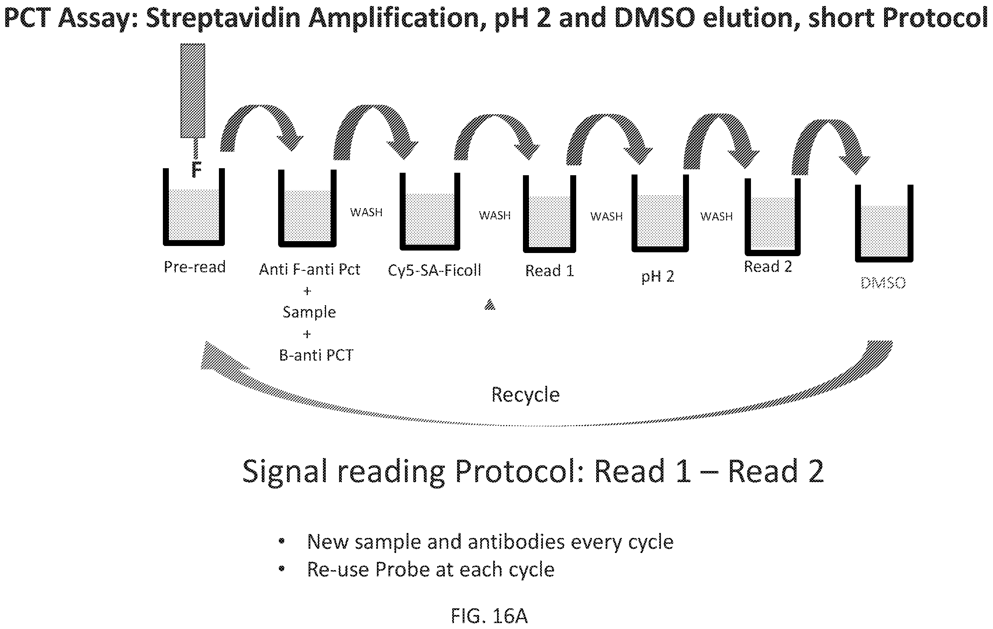

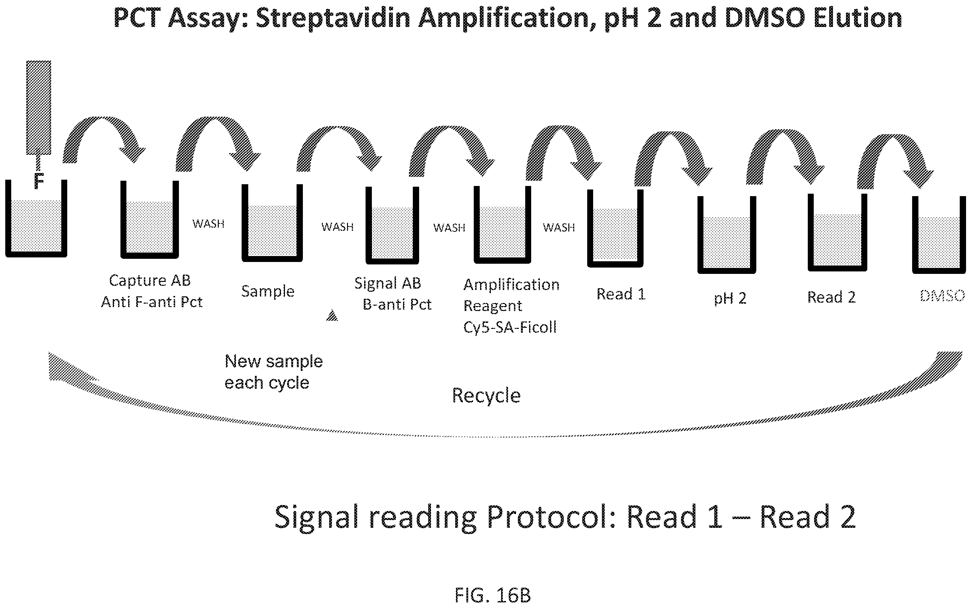

FIGs. 16A and 16B show two-read protocols, in which the fluorescent signals are read twice. The fluorescent signals are read before pH 2 acid elution as Read 1, and then read again after pH 2 acid elution as Read 2. Read 2 signal is subtracted from Read 1, and the subtracted value is quantitated against a calibration curve. The format of FIG. 16B is similar to that of FIG. 14, except that the fluorescent signals are read twice. The assay format of FIG. 16A is similar to that of FIG. 16B, except in the first binding step. In FIG. 16A, after pre-read, the sample is mixed with a dual antibody (anti-F-anti-PCT) and biotinylated-anti-PCT. The remaining Cy5-SA-FICOLL^{®} binding step and two-read steps in FIG. 16A are identical to those in FIG. 16B.
FIG. 17 shows the results of the recycle assay of 3 PCT levels (0, 10, and 100 ng/mL) by the two-read/subtraction protocol (FIG. 16B, Protocol D).
FIG. 18 shows that the results with 15 PCT clinical serum samples by two-read recycle protocol (FIG. 16B, Protocol D).
FIG. 19 shows that the results with 15 PCT clinical serum samples had high correlation (R² = 0.98) of two-read recycle protocol (FIG. 16B, Protocol D) vs. a commercial IVD instrument (Biomerieux *Vidas).*
FIG. 20 shows the results of the recycle assay of 3 PCT levels with (i) low pH plus denaturant guanidium chloride (6M guanidine.HCl, pH 1.6) elution and (ii) denaturant DMSO elution using Protocol D-1.
FIG. 21 shows the data of PCT standards with the assay format of FIG. 16A and protocol E.
FIG. 22 shows assay format of serum amyloid A (SAA), with (i) low pH plus denaturant guanidium chloride (6M guanidine.HCl, pH 1.6) elution and (ii) denaturant DMSO elution.
FIG. 23 shows the results of the recycle assay of three SAA levels with the dual low pH plus denaturant guanidium chloride elution and denaturant DMSO elution with the assay format of FIG. 22 and Protocol F.
FIG. 24 illustrates an optical configuration, for detection of fluorescence on the probe tip.

### DETAILED DESCRIPTION OF THE INVENTION

### Definitions

Terms used in the claims and specification are to be construed in accordance with their usual meaning as understood by one skilled in the art except and as defined as set forth below.

"About," as used herein, refers to within ± 10% of the recited value.

An "analyte-binding" molecule, as used herein, refers to any molecule capable of participating in a specific binding reaction with an analyte molecule. Examples include but are not limited to, (i) antigen molecules, for use in detecting the presence of antibodies specific against that antigen; (ii) antibody molecules, for use in detecting the presence of antigens; (iii) protein molecules, for use in detecting the presence of a binding partner for that protein; (iv) ligands, for use in detecting the presence of a binding partner; or (v) single stranded nucleic acid molecules, for detecting the presence of nucleic acid binding molecules.

An "aspect ratio" of a shape refers to the ratio of its longer dimension to its shorter dimension.

A "binding molecular," refers to a molecule that is capable to bind another molecule of interest.

"A binding pair," as used herein, refers to two molecules that are attracted to each other and specifically bind to each other. Examples of binding pairs include, but not limited to, an antigen and an antibody against the antigen, a ligand and its receptor, complementary strands of nucleic acids, biotin and avidin, biotin and streptavidin, lectin and carbohydrates. Preferred binding pairs are biotin and streptavidin, biotin and avidin, fluorescein and anti-fluorescein, digioxigenin/anti-digioxigenin. Biotin and avidin, including biotin derivatives and avidin derivatives such as streptavidin, may be used as intermediate binding substances in assay protocols employing complex binding sequences. For example, antibodies may be labeled with biotin ("biotinylated") and used to bind to a target substance previously immobilized on a solid phase surface. Fluorescent compositions according to the present invention employing an avidin or streptavidin may then be used to introduce the fluorescent label.

A "bispecific antibody" is an artificial protein that can simultaneously bind to two different types of antigen.

"Immobilized," as used herein, refers to reagents being fixed to a solid surface. When a reagent is immobilized to a solid surface, it is either be non-covalently bound or covalently bound to the surface.

"A monolithic substrate," as used herein, refers to a single piece of a solid material such as glass, quartz, or plastic that has one refractive index.

A "probe," as used herein, refers to a substrate coated with a thin-film layer of analyte-binding molecules at the sensing side. A probe has a distal end and a proximal end. The proximal end (also refers to probe tip in the application) has a sensing surface coated with a thin layer of analyte-binding molecules.

The present invention discloses a method to re-use an immunoassay test probe and reagents, from about 3 to 10 times, while maintaining acceptable clinical assay performance. The immunoassay test probe and reagents may be contained in one test strip, or one cartridge. The present invention re-uses test probe and reagents the and saves the cost on a per test basis.

There are several key elements to practice the invention. The first feature of this invention is that the solid phase capture reagent is coated with a hapten. Haptens, commonly defined as small organic molecules less than about 1500 Daltons, that are antigenic, but with very poor immunogenicity. Consequently, haptens have to be linked to a larger polymer, typically proteins, in order to generate anti hapten antibodies. Haptens have their antibody binding based on their primary chemical structure, and there is no conformation dependence in its antibody binding. Hapten antigens therefore would retain their antibody binding property even after repeated steps that denature anti-hapten antibodies and immunocomplexes. The present invention uses a hapten-immobilized test probe and an anti-hapten antibody to bind to the hapten-coated solid phase; the immune complex is subsequently disassociated with a denaturation reagent. Subsequently re-immersing the hapten-coated solid phase in a reagent with anti-hapten antibody restores the original amount of anti-hapten antibody to bound on the solid phase. The hapten coated solid phase can be subjected to multiple cycles of denaturation followed by anti-hapten antibody binding. The robustness of the hapten-coated solid phase allows utilization of multiple denaturation reagents for efficient elution of the immune complexes.

Suitable haptens for the present invention include, for example, small organic molecules such as nitrotyrosine, dinitrophenol, trinitrophenol, nitrophenol, and aminobenzoic acid; dyes such as Alexa Fluor, cyanine dyes, TRITC, Lucifer Yellow, Texas Red, and Bodipy; peptides such as Myc, Flag, and polyhistidine; drugs such as theophylline, phenytoin, phenobarbital, valproic acid, penicillin, and gentamycin; steroids such as progesterone, testosterone, and estradiol, and vitamins such as biotin and Vitamin D. Preferred haptens are fluorescein, biotin, and digoxigenin.

The second feature of this invention is that the assay uses a dual antibody conjugate in which the anti-hapten antibody is chemically and covalently conjugated with a capture antibody that binds to a specific analyte in a sample. The capture antibody varies depending on the analyte to be measured; this allows the assay format to be applied to many different immunoassays. The solid phase of the present invention is regenerated and recycled, while a fresh dual antibody conjugate reagent is captured on the probe at each cycle, thereby maintaining the assay performance.

The third feature of this invention is that the probe tip surface has a small dimension (≤ 5 mm in diameter) so that there is negligible consumption of the signal reagent and amplification reagent, and no replenish of those reagents is necessary during the assay cycles.

### Fluorescent Detection System

The present invention uses a fluorescent detection system as described in U.S. Patent No. 8,492,139 for measuring a fluorescent signal on a probe tip. The system comprises: (a) a probe having an aspect ratio of length to width at least 5 to 1, the probe having a first end and a second end, the second end having a sensing surface bound with a fluorescent label; (b) a light source for emitting excitation light directly to the probe's sensing surface; (c) a collecting lens pointed toward the sensing surface; and (d) an optical detector for detecting the emission fluorescent light; where the collecting lens collects and directs the emission fluorescent light to the optical detector.

The probe can be a monolithic substrate or an optical fiber. The probe can be any shape such as rod, cylindrical, round, square, triangle, etc., with an aspect ratio of length to width of at least 5 to 1, preferably 10 to 1. Because the probe is dipped in a sample solution and one or more assay solutions during an immunoassay, it is desirable to have a long probe with an aspect ratio of at least 5 to 1 to enable the probe tip's immersion into the solutions. Heterogeneous assays can be performed where the long probe is transferred to different reaction chambers. Dispensing and aspirating reagents and sample during the assay are avoided. The sensing surface of the probe is coated with analyte-binding molecules and bound with fluorescent labels.

Any light source that can emit proper excitation light for the fluorescent label is suitable for the present invention. A prefer light source is a laser that can emit light with wavelengths suitable for fluorescent labels. For example, the laser center wavelength is preferred to be 649 nm for Cy5 fluorescent dye. A suitable optical detector for detecting emission light is a photomultiplier tube (PMT), a charge coupled device (CCD), or a photodiode.

The light source and the optical detector including the collecting lens are mounted on the same side of the probe tip surface (the sensing surface). If the sensing surface faces down, they are both mounted below the tip surface. If the sensing surface faces up, they are both mounted above the tip surface. They are closer to the sensing surface than the other end of the probe. The sensing surface is always within the numeric aperture of the collecting lens. The probe can be, but it does not have to be centrally aligned with the collecting lens.

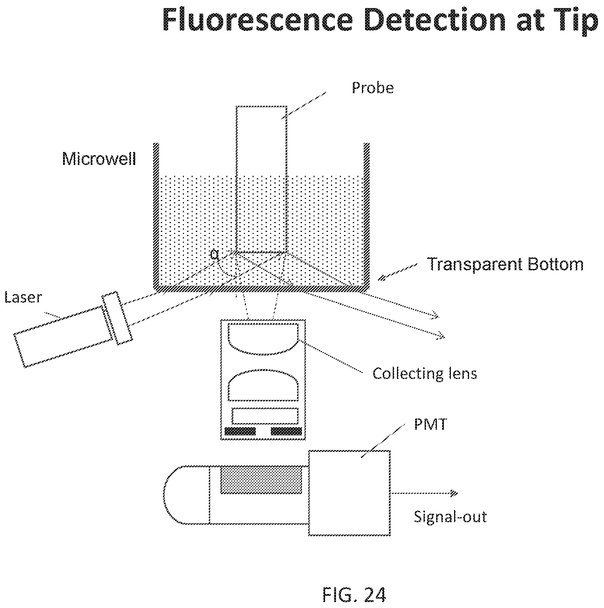

FIG. 24 illustrates an embodiment of the fluorescent detection system.

### I. Detecting an Analyte by a Recycling Protocol

The present invention is directed to a method of detecting an analyte in multiple liquid samples by a fluorescent immunoassay, using the same hapten-coated test probe, the amplification reagent, and the same signal reagent for different samples.

The method comprises the steps of: (a) dipping a probe in an aqueous solution having pH of 6.0-8.5 to pre-read the fluorescent signal of the probe tip, wherein the probe comprises a hapten immobilized on the tip of the probe, and the diameter of the tip surface is ≤ 5 mm; (b) forming an immunocomplex comprising the analyte from a sample, a capture antibody, and a signal antibody on the probe tip, wherein the capture antibody and the signal antibody are two different antibodies against the analyte, the capture antibody is covalently linked to an anti-hapten antibody against the hapten and the signal antibody is conjugated with fluorescent labels; (c) dipping the probe tip into a washing vessel containing a wash solution; (d) determining the analyte concentration in the sample by measuring the fluorescent signal of the immunocomplex at the probe tip, subtracting the pre-read fluorescent signal of (a), and quantitating the subtracted signal against a calibration curve; (e) dipping the probe tip in an acidic solution having pH about 1.0-4.0 to elute the immunocomplex from the probe tip; and (f) repeating the steps (a)-(e) except in step (b) with the analyte from a new sample, whereby the analyte in each of the multiple liquid samples is detected.

In step (e), after the probe tip is dipped in an acidic solution having pH about 1.0-4.0, optionally the probe is further dipped in a denaturant or a chaotropic agent such as dimethylsulfoxide (DMSO), n-butanol, ethanol, guanidinium chloride (guanidine hydrochloride), lithium perchlorate, lithium acetate, magnesium chloride, phenol, 2-propanol, sodium dodecyl sulfate, thiourea, and urea, to further elute the immunocomplex. DMSO is a preferred chaotropic agent.

In one embodiment, the immunocomplex of step (b) is formed by: (b1) mixing a sample solution with a dual antibody solution and a reagent solution to form a mixture, wherein the sample solution comprises the analyte, the dual antibody solution comprises the anti-hapten antibody covalently linked to the capture antibody, and the reagent solution comprises the signal antibody conjugated with fluorescent labels; and (b2) dipping the probe tip in the mixture to form the immunocomplex on the probe tip. The probe can be dipped immediately into the mixture when the components are added together. Alternatively, the probe can be dipped into the mixture after the mixture is incubated for a period of time, e.g., 30 seconds to 15 minutes. This embodiment is illustrated in FIG. 1A.

In another embodiment, the immunocomplex of step (b) is formed by: (b1) dipping the probe tip in a dual antibody solution comprising the anti-hapten antibody covalently linked to the capture antibody; (b2) dipping the probe tip in a sample solution comprising the sample; (b3) dipping the probe tip in a reagent solution comprising the signal antibody conjugated with fluorescent labels to form the immunocomplex on the probe tip. This embodiment is illustrated in FIG. 1B.

### Illustrated Embodiment 1

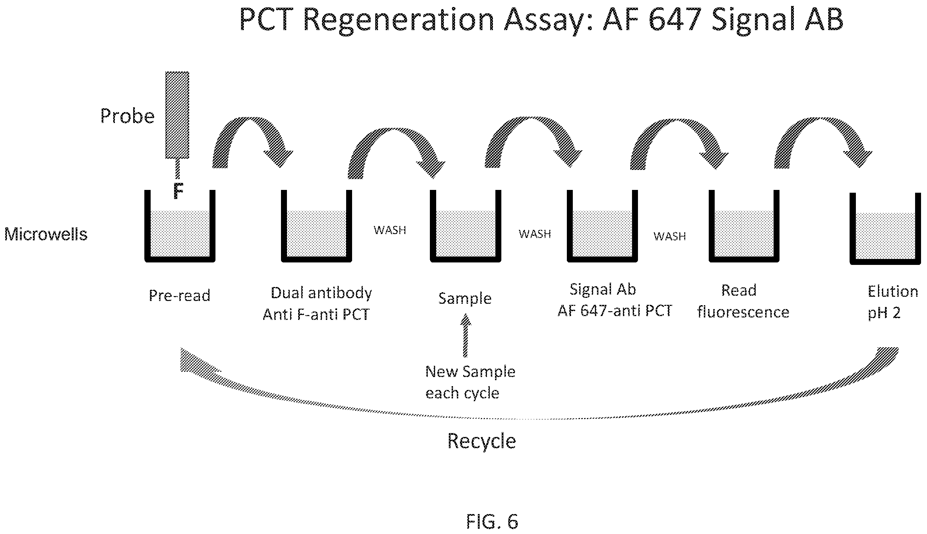

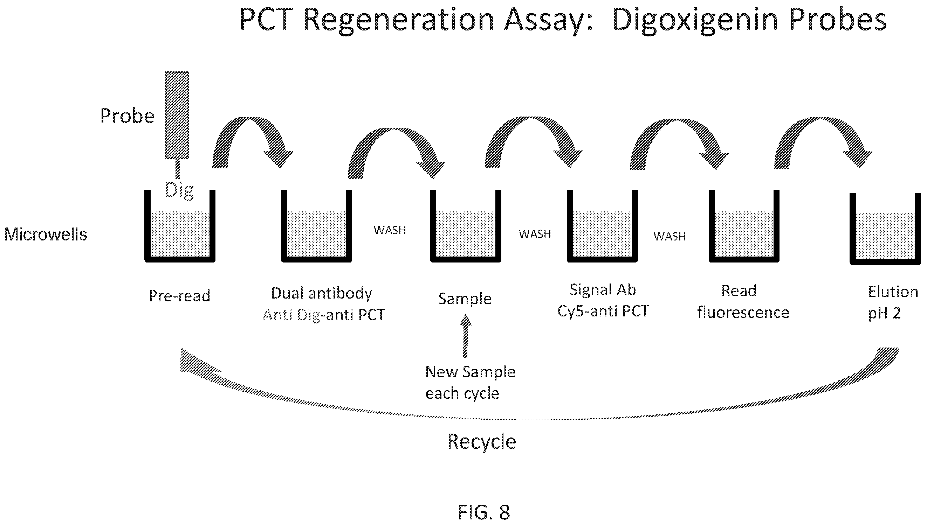

In another embodiment, the method of detecting an analyte comprises the steps of: (a) obtaining a probe having a hapten immobilized on the tip of the probe, wherein the diameter of the tip surface is ≤ 5 mm; (b) dipping the probe in an aqueous solution having pH of 6.0-8.5 to pre-read the fluorescent signal of the probe tip; (c) dipping the probe tip in a dual antibody solution comprising an anti-hapten antibody covalently linked to a capture antibody, wherein the capture antibody is a first antibody against the analyte; (d) dipping the probe tip in a sample solution comprising a liquid sample having an analyte; (e) dipping the probe tip in a reagent solution comprising a signal antibody conjugated with fluorescent labels to form an immunocomplex comprising the analyte, the capture antibody, and the signal antibody on the probe tip, wherein the signal antibody is a second antibody against the analyte; (f) dipping the probe tip into a washing vessel containing a wash solution; (g) determining the analyte concentration in the sample by measuring the fluorescent signal of the immunocomplex at the probe tip, subtracting the pre-read fluorescent signal of (b), and quantitating the subtracted signal against a calibration curve; (h) dipping the probe tip in an acidic solution having pH about 1.0-4.0 to elute the immunocomplex from the probe tip; and (i) repeating the steps (b)-(h) except in step (d) with a new sample solution comprising a new sample, whereby the analyte in each of the multiple liquid samples is detected. This embodiment is illustrated in FIGs. 1B, 6, and 8.

The method uses the same probe and the same washing solution in all cycles of reaction. Preferably, the method uses the same reagent solutions in all cycles of reaction. However, a fresh reagent solution can also be used in different cycles.

In step (a) of the present method, a probe that has a small tip for binding an analyte is obtained. The tip has a smaller surface area with a diameter ≤ 5 mm, preferably ≤ 2 mm or ≤ 1 mm. The small surface of the probe tip endows it with several advantages. In a solid phase immunoassays, having a small surface area is advantageous because it has less non-specific binding and thus produces a lower background signal. Further, the reagent or sample carry over on the probe tip is extremely small due to the small surface area of the tip. This feature makes the probe tip easy to wash, and it causes negligible contamination in the wash solution since the wash solution has a larger volume. Another aspect of the small surface area of the probe tip is that it has small binding capacity. Consequently, when the probe tip is immersed in a reagent solution, the binding of the reagent does not consume a significant amount of the reagent. The reagent concentration is effectively unchanged. Negligible contamination of the wash solution and small consumption of the reagents enable the reagents and the wash solution to be re-used many times, for example, 3-10 times or 3-20 times.

Methods to immobilize a hapten to the solid phase (the sensing surface of the probe tip) are common in immunochemistry and involve formation of covalent, hydrophobic or electrostatic bonds between the solid phase and a hapten. For example, a hapten can be conjugated to a carrier protein and the hapten-protein is immobilized either by adsorption to the solid surface or by covalently binding to aminopropylsilane coated on the solid surface.

In step (b), the fluorescent signal of the probe tip is pre-read by a fluorescent detection system in a read vessel (or a read chamber, or a read well). The read vessel contains an aqueous solution such as water or a buffer having pH between 6.0 to 8.5. Preferably, the aqueous solution contains 1-10 mM or 1-100 mM of phosphate buffer, tris buffer, citrate buffer or other buffer suitable for pH between 6.0-8.5, to neutralize the probe after low pH regeneration.

Pre-read is necessary before the first sample binding to establish a baseline of any potential background fluorescence for the first cycle reaction. Pre-read is also necessary after the regeneration of the probe tip and before the next sample binding to establish a baseline for subsequent cycles. After each cycle, the pre-read signal is preferably the same. If the pre-read signal is higher, or lower than the pre-read signal of the previous cycle, subtracting the pre-read fluorescent signal from the measured fluorescent signal after the immunoassay in each cycle, and then quantitating the subtracted signal against a calibration curve would still provide a correct quantitation of the analyte.

In step (c) of the method, the probe tip is dipped into a dual antibody vessel containing a dual antibody solution. The dual antibody solution contains an anti-hapten antibody covalently linked to a capture antibody, wherein the capture antibody is a first antibody against the analyte. Alternatively, the dual antibody solution contains a bi-specific antibody that simultaneously bind to the hapten and the analyte.

In one embodiment, the anti-hapten antibody and the capture antibody are directly linked to each other without a linker.

In a preferred embodiment, the anti-hapten antibody and the capture antibody are both covalently linked to a polymer, which serves as a linker or spacer. The polymer in general has a molecular weight of 1,000 to 500,000 Daltons. The polymer can be a polysaccharide (e.g., dextran, amylose), a dendrimer, or a polyethylene glycol. In one preferred embodiment, the polymer is FICOLL^{®} (copolymers of sucrose and epichlorohydrin).

In step (d) of the method, the probe tip is dipped into a sample vessel (or a sample chamber or a sample well), and incubated for 5 seconds to 5 minutes, 10 seconds to 2 minutes, or 30 seconds to 1 minute, to bind the analyte to the first antibody on the probe tip.

After step (d), the probe is optionally washed 1-5 times, preferably 1-3 times in a wash vessel (or a wash chamber or a wash well) containing a wash solution. This extra washing step may not be required because the amount of the carried-over solution is minimal due to a small binding surface area. The wash solution typically contains buffer and a surfactant such as Tween 20.

In step (e) of the method, the probe tip is dipped into a reagent vessel (or a reagent chamber or a reagent sell) for 5 seconds to 5 minutes, 10 seconds to 2 minutes, or 30 seconds to 1 minute, to bind the reagent to the analyte on the probe tip. The reagent solution comprises a fluorescent labelled second antibody (a signal antibody). Any suitable fluorescent label can be used in this method. An example of a fluorescent label is an arylsulfonate cyanine fluorescent dye as described in Mujumdar et al. (1993) Bioconjugate Chemistry, 4:105-111; Southwick et al. (1990) Cytometry, 11:418-430; and U.S. Pat. No. 5,268,486. Cy5 is a preferred arylsulfonate cyanine fluorescent dye, because it has a high extinction coefficient and good quantum yield; it also has fluorescent emission spectra in a range (500 nm to 750 nm) outside of the autofluorescence wavelengths of most biological materials and plastics. In addition, Cy5 has a good solubility in water, and has low non-specific binding characteristics.

A fluorescent label can covalently bind to the signal antibody through a variety of moieties, including disulfide, hydroxyphenyl, amino, carboxyl, indole, or other functional groups, using conventional conjugation chemistry as described in the scientific and patent literature. Exemplary techniques for binding arylsulfonate cyanine fluorescent dye labels to antibodies and other proteins are described in U.S. Pat. Nos. 5,268,486; 5,650,334. Techniques for linking a preferred Cy5 fluorescent label to antibodies are described in a technical bulletin identified as Cat. No. A25000, published by Biological Detection Systems, Inc., Pittsburgh, Pa.

In Step (f), the probe is washed 1-5 times, preferably 1-3 times in a wash vessel containing a wash solution. The wash solution typically contains a buffer and a surfactant such as Tween 20.

In step (g), the probe stays in the wash vessel or is moved to a measurement vessel and the fluorescent signal of the bound immunocomplex is detected by the fluorescent detection system as described above, where the light source and the detector are mounted at the same side (the proximal side) of the sensing surface of the probe. The measurement vessel can be a separate well or can be the same pre-read vessel.

Alternatively, the methods of the present invention can be detected by other suitable fluorescent detection systems.

The analyte concentration in the sample is determined by measuring the fluorescent signal of the immunocomplex at the probe tip, subtracting the pre-read fluorescent signal of (b), and then quantitating against a calibration curve (a standard curve).

The calibration curve is typically pre-established before assaying the samples according to the methods known to a person skilled in the art. In a preferred embodiment, the fluorescent signals (after subtracting the pre-read signal) of the same sample remain constant at each cycle, and the calibration curves are the same for each cycle.

In another embodiment, the fluorescent signals (after subtracting the pre-read signal) of the same sample increase or decrease at each cycle, and a cycle-specific calibration curve needs to be established for each cycle. In these instances with changes in fluorescent signals, samples are quantitated against a cycle-specific calibration curve, and the quantitated results can still be consistent in spite of the increase or decrease of the fluorescent signals at different cycles.

In step (h), the probe is regenerated by employing a denaturing condition that dissociates the immune complexes bound to the capture antibody on a solid phase. An acid or an acidic buffer having pH about 1 to about 4 is effective to regenerate the antibody probe of the present invention. For example, hydrochloric acid, sulfuric acid, nitric acid, acetic acid can be used to regenerate the probe. The probe is first treated with an acidic condition, and then neutralized by a neutral aqueous solution such as a buffer having pH between 6.0-8.5. In one embodiment, the low pH treated probe is conveniently neutralized in the read vessel of step (b) before pre-read. Alternatively, the low pH treated probe can be neutralized in a separate vessel having a buffer with a pH of 6.0-8.5. The regeneration procedures can be one single acidic treatment, followed by neutralization. For example, a single pH 1-3, or pH 1.5-2.5 (e.g., pH 2) exposure ranging from 10 seconds to 2 minutes is effective. The regeneration procedures can also be a "pulse" regeneration step, where the probe is exposed to 2-5 cycles (e.g. 3 cycles) of a short pH treatment (e.g., 10-20 seconds), followed by neutralization at pH 6.5-8.0 (e.g., 10-20 seconds).

After regeneration of the probe, steps of (b)-(h) are repeated with a different sample in a subsequent cycle, for 1-10, 1-20, 1-25, 3-20, 5-10, 5-20, 5-25, or 5-30 times, with the same probe and the same reagents. In each cycle, the regenerated probe has fresh dual antibody captured on the probe.

In one embodiment, the reaction is accelerated by agitating or mixing the solution in the vessel. For example, a flow such as a lateral flow or an orbital flow of the solution across the probe tip can be induced in one or more reaction vessels, including sample vessel, reagent vessel, wash vessels, and regeneration vessel, to accelerates the binding reactions, dissociation. For example, the reaction vessels can be mounted on an orbital shaker and the orbital shaker is rotated at a speed at least 50 rpm, preferably at least 200 rpm or at least 500 rpm, such as 50-200 or 500-1,500 rpm. Additionally, the probe tip can be moved up and down and perpendicular to the plane of the orbital flow, at a speed of 0.01 to 10 mm/second, in order to induce additional mixing of the solution above and below the probe tip.

### Illustrated Method 2 (not covered by the claimed invention)

In a second method for detecting an analyte (which is not covered by the claimed invention), the method is similar to that of the first embodiment except steps (b) and (c) are reversed in order. The method of the second embodiment comprises the steps of: (a) obtaining a probe having a hapten immobilized on the tip of the probe, wherein the diameter of the tip surface is ≤ 5 mm; (b) dipping the probe tip in a dual antibody solution comprising a dual antibody comprising an anti-hapten antibody covalently linked to a capture antibody, wherein the capture antibody is a first antibody against the analyte;
(c) dipping the probe in an aqueous solution having pH of 6.0-8.5 to pre-read the fluorescent signal of the probe tip; (d) dipping the probe tip in a sample solution comprising a liquid sample having an analyte; (e) dipping the probe tip in a reagent solution comprising a signal antibody conjugated with fluorescent labels to form an immunocomplex comprising the analyte, the capture antibody, and the signal antibody on the probe tip, wherein the signal antibody is a second antibody against the analyte; (f) dipping the probe tip in a wash solution; (g) determining the analyte concentration in the sample by measuring the fluorescent signal of the immunocomplex at the probe tip, subtracting the pre-read fluorescent signal of (c), and quantitating the subtracted signal against a calibration curve; (h) dipping the probe tip in an acidic solution having pH about 1.0-4.0 to elute the immunocomplex from the probe tip; and
(i) repeating the steps (b)-(h) except in step (d) with a new sample solution containing a new sample, whereby the analyte in each of the multiple liquid samples is detected.

The details of each step are similar to those described above in the first embodiment.

In the second method, the dual antibody conjugate optionally comprises a second fluorescent label that is different from the signal fluorescent label in the excitation wavelength and emission wavelength. The pre-read of step (b) determines the signal of the second fluorescent label, which shows whether the recycled probe still provides an acceptable binding of the dual antibody conjugate to the hapten-immobilized probe tip after reacting with multiple samples. If the probe is masked by interfering materials after multiple sample reaction, the pre-read of a second fluorescent signal in step (b) will show a significant decrease in the signal of the second fluorescent label, and the assay should be repeated with a fresh probe.

### II. Amplification with a Second Hapten and Anti-Second Hapten Antibody or Streptavidin

The methods described below add amplification steps to the Recycling Protocol I, by amplifying the fluorescent signals with a second hapten and an anti-second hapten antibody or streptavidin. The second hapten is different from the hapten immobilized on the probe tip. The details of each step are the same or similar to those of the corresponding steps described above in the Recycling Protocol I.

The method comprises the steps of: (a) dipping a probe in an aqueous solution having pH of 6.0-8.5 to pre-read the fluorescent signal of the probe tip, wherein the probe comprises a first hapten immobilized on the tip of the probe, and the diameter of the tip surface is ≤ 5 mm; (b) forming a first immunocomplex comprising the analyte from a sample, a capture antibody, and a signal antibody on the probe tip, wherein the capture antibody and the signal antibody are two different antibodies against the analyte, the capture antibody is covalently linked to an anti-first hapten antibody against the first hapten and the signal antibody is conjugated with a second hapten, the first hapten and the second hapten are different; (c) dipping the probe tip in a wash solution; (d) dipping the probe tip in an amplification solution comprising (i) an anti-second hapten antibody or (ii) streptavidin, conjugated to fluorescent labels to form a second immunocomplex comprising the analyte, the capture antibody, the signal antibody, the second hapten, and the anti-second hapten antibody or streptavidin on the probe tip; (e) determining the analyte concentration in the sample by measuring the fluorescent signal of the second immunocomplex at the probe tip, subtracting the pre-read fluorescent signal of (a), and quantitating the subtracted signal against a calibration curve; (f) dipping the probe tip in an acidic solution having pH about 1.0-4.0 to elute the immunocomplexes from the probe tip; and (g) repeating the same steps (a)-(f) except in step (b) with the analyte from a new sample, whereby the analyte in each of the multiple liquid samples is detected.

In one embodiment, the first immunocomplex of step (b) is formed by: (b1) mixing a sample solution with a dual antibody solution and a reagent solution to form a mixture, wherein the sample solution comprises the analyte, the dual antibody solution comprises the anti-first hapten antibody covalently linked to the capture antibody, and the reagent solution comprises the signal antibody conjugated with the second hapten; and (b2) dipping the probe tip in the mixture to form the first immunocomplex on the probe tip.

In another embodiment, the first immunocomplex of step (b) is formed by: (b1) dipping the probe tip in a dual antibody solution comprising the anti-first hapten antibody covalently linked to the capture antibody; (b2) dipping the probe tip in a sample solution comprising the sample; and (b3) dipping the probe tip in a reagent solution comprising the signal antibody conjugated with the second hapten to form the first immunocomplex on the probe tip.

### IIA. Amplification with Second Hapten and Anti-Second Hapten Antibody

In this protocol, the fluorescent signals are amplified with a second hapten and an anti-second hapten antibody.

The method comprises the steps of: (a) dipping a probe in an aqueous solution having pH of 6.0-8.5 to pre-read the fluorescent signal of the probe tip, wherein the probe comprises a first hapten immobilized on the tip of the probe, and the diameter of the tip surface is ≤ 5 mm; (b) forming a first immunocomplex comprising the analyte from a sample, a capture antibody, and a signal antibody on the probe tip, wherein the capture antibody and the signal antibody are two different antibodies against the analyte, the capture antibody is covalently linked to an anti-first hapten antibody against the first hapten and the signal antibody is conjugated with a second hapten, the first hapten and the second hapten are different; (c) dipping the probe tip in a wash solution; (d) dipping the probe tip in an amplification solution comprising an anti-second hapten antibody conjugated to fluorescent labels to form a second immunocomplex comprising the analyte, the capture antibody, the signal antibody, the second hapten, and the anti-second hapten antibody on the probe tip; (e) determining the analyte concentration in the sample by measuring the fluorescent signal of the second immunocomplex at the probe tip, subtracting the pre-read fluorescent signal of (a), and quantitating the subtracted signal against a calibration curve; (f) dipping the probe tip in an acidic solution having pH about 1.0-4.0 to elute the immunocomplexes from the probe tip; and (g) repeating the same steps (a)-(f) except in step (b) with the analyte from a new sample, whereby the analyte in each of the multiple liquid samples is detected.

In step (f), the probe tip is dipped in an acidic solution having pH about 1.0-4.0, optionally, the acidic solution may comprise a denaturant or a chaotropic agent. Optionally, the probe may be further dipped in a denaturant or a chaotropic agent. A denaturant or a chaotropic agent for example includes dimethylsulfoxide (DMSO), n-butanol, ethanol, guanidinium chloride, lithium perchlorate, lithium acetate, magnesium chloride, phenol, 2-propanol, sodium dodecyl sulfate, thiourea, and urea, to further elute the immunocomplex. DMSO and guanidinium chloride are preferred denaturants or chaotropic agents.

In one embodiment, the first immunocomplex of step (b) is formed by: (b1) mixing a sample solution with a dual antibody solution and a reagent solution to form a mixture, wherein the sample solution comprises the analyte, the dual antibody solution comprises the anti-first hapten antibody covalently linked to the capture antibody, and the reagent solution comprises the signal antibody conjugated with the second hapten; and (b2) dipping the probe tip in the mixture to form the first immunocomplex on the probe tip.

In another embodiment, the first immunocomplex of step (b) is formed by: (b1) dipping the probe tip in a dual antibody solution comprising the anti-first hapten antibody covalently linked to the capture antibody; (b2) dipping the probe tip in a sample solution comprising the sample; and (b3) dipping the probe tip in a reagent solution comprising the signal antibody conjugated with the second hapten to form the first immunocomplex on the probe tip.

### Illustrated Embodiment 1

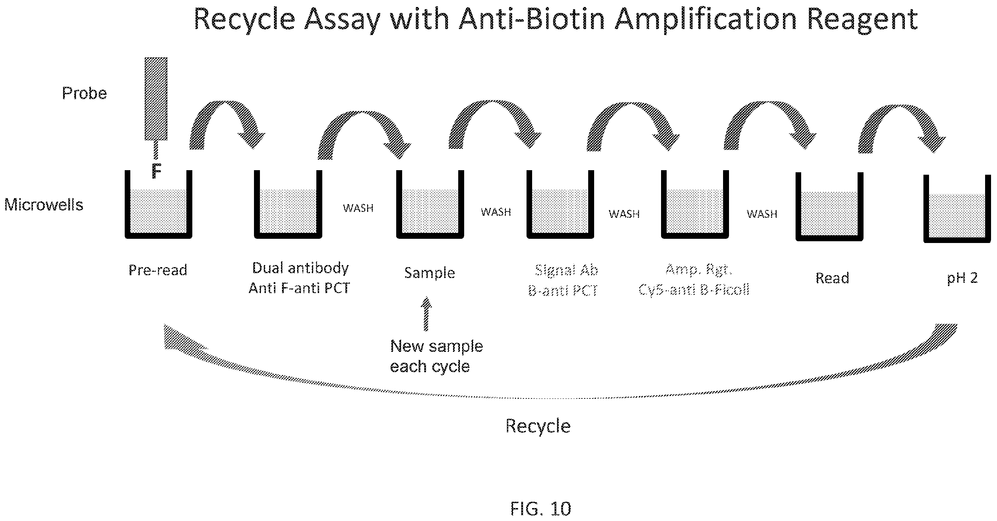

For example, the method comprises the steps of: (a) obtaining a probe having a first hapten immobilized on the tip of the probe, wherein the diameter of the tip surface is ≤ 5 mm; (b) dipping the probe an aqueous solution having pH of 6.0-8.5 to pre-read the fluorescent signal of the probe tip; (c) dipping the probe tip in a dual antibody solution comprising an anti-first hapten antibody covalently linked to a capture antibody, wherein the capture antibody is a first antibody against the analyte; (d) dipping the probe tip in a sample solution comprising a liquid sample having an analyte; (e) dipping the probe tip in a reagent solution comprising a signal antibody conjugated with a second hapten to form a first immunocomplex comprising the analyte, the capture antibody, and the signal antibody on the probe tip, wherein the signal antibody is a second antibody against the analyte and the first hapten and the second hapten are different; (f) dipping the probe tip in a wash solution; (g) dipping the probe tip in an amplification solution comprising an anti-second hapten antibody conjugated to fluorescent labels to form a second immunocomplex comprising the analyte, the capture antibody, the signal antibody, the second hapten, and the anti-second hapten antibody on the probe tip; (h) determining the analyte concentration in the sample by measuring the fluorescent signal of the second immunocomplex at the probe tip, subtracting the pre-read fluorescent signal of (b), and quantitating the subtracted signal against a calibration curve; (i) dipping the probe tip in an acidic solution having pH about 1.0-4.0 to elute the immunocomplexes from the probe tip; and (j) repeating the same steps (b)-(i) except in step (d) with a new sample solution containing a new sample, whereby the analyte in each of the multiple liquid samples is detected. This embodiment is illustrated in FIG. 10, where biotin is a second hapten.

### Illustrated Method 2 (not covered by the claimed invention)

In this method (which is not covered by the claimed invention), the method comprises the steps of: (a) obtaining a probe having a first hapten immobilized on the tip of the probe, wherein the diameter of the tip surface is ≤ 5 mm; (b) dipping the probe tip in a dual antibody solution comprising a dual antibody comprising an anti-hapten antibody covalently linked to a capture antibody, wherein the capture antibody is a first antibody against the analyte; (c) dipping the probe in an aqueous solution having pH of 6.0-8.5 to pre-read the fluorescent signal of the probe tip; (d) dipping the probe tip in a sample solution comprising a liquid sample having an analyte; (e) dipping the probe tip in a reagent solution comprising a signal antibody conjugated with a second hapten to form a first immunocomplex comprising the analyte, the capture antibody, and the signal antibody on the probe tip, wherein the signal antibody is a second antibody against the analyte and the first hapten and the second hapten are different; (f) dipping the probe tip in a wash solution; (g) dipping the probe tip in an amplification solution comprising an anti-second hapten antibody conjugated to fluorescent labels to form a second immunocomplex comprising the analyte, the capture antibody, the signal antibody, the second hapten, and the anti-second hapten antibody on the probe tip; (h) determining the analyte concentration in the sample by measuring the fluorescent signal of the second immunocomplex at the probe tip, subtracting the pre-read fluorescent signal of (c), and quantitating the subtracted signal against a calibration curve; (i) dipping the probe tip in an acidic solution having pH about 1.0-4.0 to elute the immunocomplexes from the probe tip; and (j) repeating the same steps (b)-(i) except in step (d) with a new sample solution containing a new sample, whereby the analyte in each of the multiple liquid samples is detected.

In both amplification methods, the anti-second hapten antibody and the fluorescent label conjugate may be further conjugated to FICOLL^{®} to increase the loading of anti-second hapten antibody and the fluorescent labels.

### IIB. Amplification with Biotin and Streptavidin

The methods described below add amplification steps to the Recycling Protocol I, by amplifying the fluorescent signals with biotin and streptavidin. In these methods, the hapten on the probe tip is a non-biotin hapten. Using of the streptavidin amplification reagent demands more stringent elution conditions, and dimethyl sulfoxide (DMSO) is used as a second elution agent after the acidic elution.

The method comprises the steps of: (a) dipping a probe in an aqueous solution having pH of 6.0-8.5 to pre-read the fluorescent signal of the probe tip, wherein the probe comprises a non-biotin hapten immobilized on the tip of the probe, and the diameter of the tip surface is ≤ 5 mm; (b) forming a first immunocomplex comprising the analyte from a sample, a capture antibody, and a signal antibody on the probe tip, wherein the capture antibody and the signal antibody are two different antibodies against the analyte, the capture antibody is covalently linked to an anti-hapten antibody and the signal antibody is conjugated with biotin; (c) dipping the probe tip in a wash solution; (d) dipping the probe tip in an amplification solution comprising streptavidin conjugated to fluorescent labels to form a second immunocomplex comprising the analyte, the capture antibody, the signal antibody, the biotin, and streptavidin on the probe tip; (e) determining the analyte concentration in the sample by measuring the fluorescent signal of the second immunocomplex at the probe tip, subtracting the pre-read fluorescent signal of (a), and quantitating the subtracted signal against a calibration curve; (f) dipping the probe tip in an acidic solution having pH about 1.0-4.0 to elute the immunocomplexes from the probe tip; and (g) repeating the same steps (a)-(f) except in step (b) with the analyte from a new sample, whereby the analyte in each of the multiple liquid samples is detected.

In step (f), after the probe tip is dipped in an acidic solution having pH about 1.0-4.0, optionally the probe is further dipped in a denaturant or a chaotropic agent such as dimethylsulfoxide (DMSO), n-butanol, ethanol, guanidinium chloride, lithium perchlorate, lithium acetate, magnesium chloride, phenol, 2-propanol, sodium dodecyl sulfate, thiourea, and urea, to further elute the immunocomplex. DMSO is a preferred chaotropic agent.

In one embodiment, the first immunocomplex of step (b) is formed by: (b1) mixing a sample solution with a dual antibody solution and a reagent solution to form a mixture, wherein the sample solution comprises the analyte, the dual antibody solution comprises the anti-hapten antibody covalently linked to the capture antibody, and the reagent solution comprises the signal antibody conjugated with biotin; and (b2) dipping the probe tip in the mixture to form the first immunocomplex on the probe tip.

In another embodiment, the immunocomplex of step (b) is formed by: (b1) dipping the probe tip in a dual antibody solution comprising the anti-hapten antibody covalently linked to the capture antibody; (b2) dipping the probe tip in a sample solution comprising the sample; and (b3) dipping the probe tip in a reagent solution comprising the signal antibody conjugated with the biotin to form the first immunocomplex on the probe tip.

### Illustrated Embodiment 1

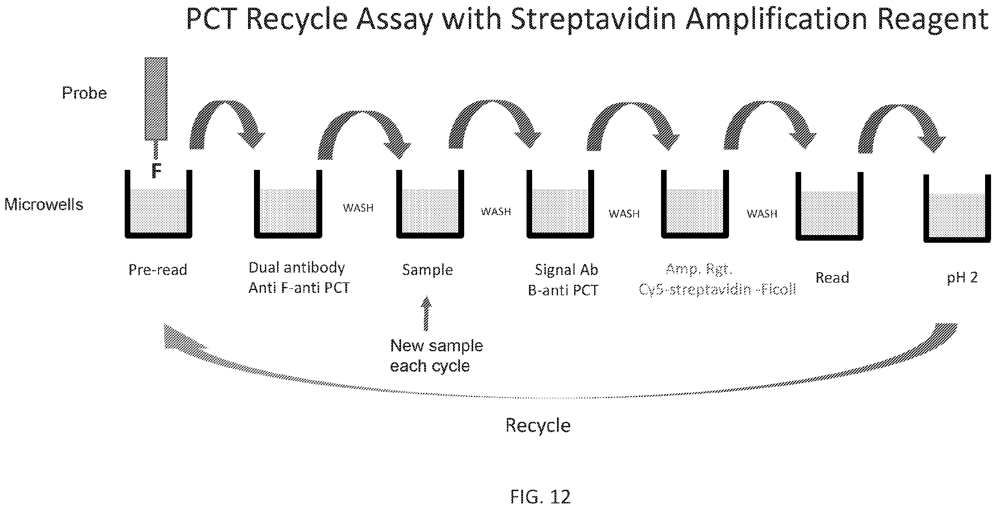

In one embodiment, the method comprises the steps of: (a) obtaining a probe having a hapten immobilized on the tip of the probe, wherein the diameter of the tip surface is ≤ 5 mm, and the hapten is not biotin; (b) dipping the probe in an aqueous solution having pH of 6.0-8.5 to pre-read the fluorescent signal of the probe tip; (c) dipping the probe tip in a dual antibody solution comprising an anti-hapten antibody covalently linked to a capture antibody, wherein the capture antibody is an antibody against the analyte; (d) dipping the probe tip in a sample solution containing a liquid sample having an analyte; (e) dipping the probe tip into a reagent solution comprising a signal antibody conjugated with biotin to form a first immunocomplex comprising the analyte, the capture antibody, and the signal antibody on the probe tip, wherein the signal antibody is a second antibody against the analyte; (f) dipping the probe tip in a wash solution; (g) dipping the probe tip in an amplification solution comprising streptavidin conjugated to fluorescent labels to form a second immunocomplex comprising the analyte, the capture antibody, the signal antibody, the biotin, and the streptavidin on the probe tip, (h) determining the analyte concentration in the sample by measuring the fluorescent signal of the second immunocomplex at the probe tip, subtracting the pre-read fluorescent signal of (b), and quantitating the subtracted signal against a calibration curve; (i) dipping the probe tip in an acidic solution having pH about 1.0-4.0, optionally further in a chaotropic agent such as dimethyl sulfoxide to elute the immunocomplexes from the probe tip; and (j) repeating the same steps (b)-(i) except in step (d) with a new sample solution comprising a new sample, whereby the analyte in each of the multiple liquid samples is detected. This embodiment is illustrated in FIG. 12 and 14.

### Illustrated Method 2 (not covered by the claimed invention)

In another method (which is not covered by the claimed invention), the method comprises the steps of: (a) obtaining a probe having a hapten immobilized on the tip of the probe, wherein the diameter of the tip surface is ≤ 5 mm, and the hapten is not biotin; (b) dipping the probe tip into a dual antibody vessel containing a dual antibody solution comprising an anti-hapten antibody covalently linked to a capture antibody, wherein the capture antibody is an antibody against the analyte; (c) dipping the probe in a pre-read vessel comprising an aqueous solution having pH of 6.0-8.5 to pre-read the fluorescent signal of the probe tip; (d) dipping the probe tip in a sample solution containing a liquid sample having an analyte; (e) dipping the probe tip into a reagent solution comprising a signal antibody conjugated with biotin to form a first immunocomplex comprising the analyte, the capture antibody, and the signal antibody on the probe tip, wherein the signal antibody is a second antibody against the analyte; (f) dipping the probe tip in a wash solution; (g) dipping the probe tip in an amplification solution comprising streptavidin conjugated to fluorescent labels to form a second immunocomplex comprising the analyte, the capture antibody, the signal antibody, the biotin, and the streptavidin on the probe tip, (h) determining the analyte concentration in the sample by measuring the fluorescent signal of the second immunocomplex at the probe tip, subtracting the pre-read fluorescent signal of (b), and quantitating the subtracted signal against a calibration curve; (i) dipping the probe tip first in an acidic solution having pH about 1.0-4.0, optionally further in a chaotropic agent such as dimethyl sulfoxide to elute the immunocomplexes from the probe tip; and (j) repeating the same steps (b)-(i) except in step (d) with a new sample solution comprising a new sample, whereby the analyte in each of the multiple liquid samples is detected.

In the amplification of the second method, the dual antibody conjugate optionally comprises a second fluorescent label that is different from the signal fluorescent label in the excitation wavelength and emission wavelength.

In both amplification methods, the streptavidin and the fluorescent label conjugate may be further conjugated to FICOLL^{®} to increase the loading of streptavidin and the fluorescent labels.

### III. Two-Read Protocol (not covered by the claimed invention)

The two-read protocol (which is not covered by the claimed invention) reads a first fluorescent signal of the second immunocomplex at the probe tip, then reads a second fluorescent signal of the acid-treated probe tip, and then subtracts the second fluorescent signal from the first fluorescent signal to quantitate the subtracted signal against a calibration curve. With this two-read and subtraction method, the background fluorescent signal remains low for more than 10 cycles. Low background improves the detection of sample having low concentration and improves the sensitivity of the assay.

The details of each step are the same or similar to those of the corresponding steps described above in the recycling protocol I.

The method comprises the steps of: (a) obtaining a probe having a non-biotin hapten immobilized on the tip of the probe, wherein the diameter of the tip surface is ≤ 5 mm; (b) forming a first immunocomplex comprising the analyte from a sample, a capture antibody, and a signal antibody on the probe tip, wherein the capture antibody and the signal antibody are two different antibodies against the analyte, the capture antibody is covalently linked to an anti-hapten antibody and the signal antibody is conjugated with biotin; (c) dipping the probe tip in a wash solution; (d) dipping the probe tip in an amplification solution comprising streptavidin conjugated to fluorescent labels to form a second immunocomplex comprising the analyte, the capture antibody, the signal antibody, the biotin, and streptavidin on the probe tip; (e) reading a first fluorescent signal of the second immunocomplex at the probe tip; (f) dipping the probe tip in an acidic solution having pH about 1.0-4.0; (g) reading a second fluorescent signal of the acid-treated probe tip, (h) subtracting the second fluorescent signal from the first fluorescent signal, and quantitating the subtracted signal against a calibration curve; (i) dipping the probe tip in a chaotropic agent such as dimethyl sulfoxide; and (j) repeating the same steps (b)-(i) except in step (b) with a new sample solution comprising a new sample, whereby the analyte in each of the multiple liquid samples is detected.

In one example, the first immunocomplex of step (b) is formed by: (b1) mixing a sample solution with a dual antibody solution and a reagent solution to form a mixture, wherein the sample solution comprises the analyte, the dual antibody solution comprises the anti-hapten antibody covalently linked to the capture antibody, and the reagent solution comprises the signal antibody conjugated with biotin; and(b2) dipping the probe tip in the mixture to form the first immunocomplex on the probe tip. This is illustrated in FIG. 16A.

In another example, the immunocomplex of step (b) is formed by: (b1) dipping the probe tip in a dual antibody solution comprising the anti-hapten antibody covalently linked to the capture antibody; (b2) dipping the probe tip in a sample solution comprising the sample; and (b3) dipping the probe tip in a reagent solution comprising the signal antibody conjugated with the biotin to form the first immunocomplex on the probe tip. This is illustrated in FIG. 16B.

For example, the method comprises the steps of: (a) obtaining a probe having a hapten immobilized on the tip of the probe, wherein the diameter of the tip surface is ≤ 5 mm, and the hapten is not biotin; (b) dipping the probe tip in a dual antibody solution comprising an anti-hapten antibody covalently linked to a capture antibody, wherein the capture antibody is an antibody against the analyte; (c) dipping the probe tip in a sample solution containing a liquid sample having an analyte; (d) dipping the probe tip into a reagent solution comprising a signal antibody conjugated with biotin to form a first immunocomplex comprising the analyte, the capture antibody, and the signal antibody on the probe tip, wherein the signal antibody is a second antibody against the analyte; (e) dipping the probe tip in a wash solution; (f) dipping the probe tip in an amplification solution comprising streptavidin conjugated to fluorescent labels to form a second immunocomplex comprising the analyte, the capture antibody, the signal antibody, the biotin, and the streptavidin on the probe tip, (g) reading a first fluorescent signal of the second immunocomplex at the probe tip; (h) dipping the probe tip in an acidic solution having pH about 1.0-4.0; (i) reading a second fluorescent signal of the acid-treated probe tip; (j) subtracting the second fluorescent signal from the first fluorescent signal, and quantitating the subtracted signal against a calibration curve; (k) dipping the probe tip in a chaotropic agent such as dimethyl sulfoxide; and (j) repeating the same steps (b)-(k) except in step (c) with a new sample solution comprising a new sample, whereby the analyte in each of the multiple liquid samples is detected. This is illustrated in FIG. 16B.

In step (h), the acidic solution may further comprise a denaturant or a chaotropic agent, for example, guanidium chloride, in a concentration 2M-8M, or 5-7M, e.g., 6M.

In the two-read protocol, pre-read of a fluorescent signal of the probe prior to dipping the probe in a sample solution in each cycle is optional. The probe can be pre-read either before step (b), or after step (b) but before step (c).

In the two-read protocol, streptavidin and the fluorescent label conjugate may be further conjugated to FICOLL^{®} to increase the loading of streptavidin and the fluorescent labels.

### IV. Dual Denaturant Protocol

For some proteins that are sticky or tend to aggregate, using two different denaturants help to remove the immunocomplex formed on the probe after each cycle of immunoreaction. The inventors have discovered that elution using guanidine hydrochloride (guanidium chloride) in acidic pH (pH 1-4 or 1.5 to 2.5), followed by elution with DMSO are effective to remove the bound immunocomplex after each cycle and provide a regenerated probe with a good performance.

In one embodiment, the method comprises the step of: (a) dipping a probe in an aqueous solution having pH of 6.0-8.5 to pre-read the fluorescent signal of the probe tip, wherein the probe comprises a hapten immobilized on the tip of the probe, and the diameter of the tip surface is ≤ 5 mm; (b) forming an immunocomplex comprising the analyte from a sample, a capture antibody, and a signal antibody on the probe tip, wherein the capture antibody and the signal antibody are two different antibodies against the analyte, the capture antibody is covalently linked to an anti-hapten antibody against the hapten and the signal antibody is conjugated with fluorescent labels; (c) dipping the probe tip into a washing vessel containing a wash solution; (d) reading a first fluorescent signal of the immunocomplex at the probe tip; (e) dipping the probe tip in an acidic solution having pH about 1.0-4.0 and containing guanidium chloride; (f) reading a second fluorescent signal of the acid-treated probe tip, (g) subtracting the second fluorescent signal from the first fluorescent signal, and quantitating the subtracted signal against a calibration curve; (h) dipping the probe tip in a chaotropic agent such as dimethyl sulfoxide; and (i) repeating the same steps (b)-(h) except in step (b) with a new sample solution comprising a new sample, whereby the analyte in each of the multiple liquid samples is detected.

In one embodiment, the immunocomplex of step (b) is formed by: (b1) mixing a sample solution with a dual antibody solution and a reagent solution to form a mixture, wherein the sample solution comprises the analyte, the dual antibody solution comprises the anti-hapten antibody covalently linked to the capture antibody, and the reagent solution comprises the signal antibody conjugated with fluorescent labels; and (b2) dipping the probe tip in the mixture to form the immunocomplex on the probe tip. The probe can be dipped immediately into the mixture when the components are added together. Alternatively, the probe can be dipped into the mixture after the mixture is incubated for a period of time, e.g., 30 seconds to 15 minutes.

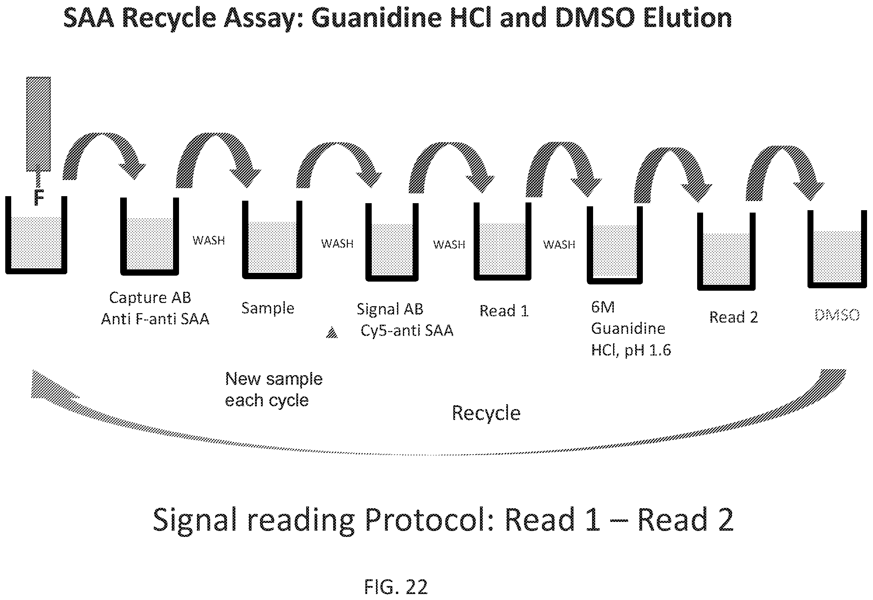

In another embodiment, the immunocomplex of step (b) is formed by: (b1) dipping the probe tip in a dual antibody solution comprising the anti-hapten antibody covalently linked to the capture antibody; (b2) dipping the probe tip in a sample solution comprising the sample; (b3) dipping the probe tip in a reagent solution comprising the signal antibody conjugated with fluorescent labels to form the immunocomplex on the probe tip. This embodiment is illustrated in FIG. 22.

In step (e), the guanidium chloride concentration in general is 2-8 M, or 3-7 M, or 4-6 M, e.g., 6 M.

The details of each step are the same or similar to those of the corresponding steps described above in the Recycling Protocols I-III.

In all the methods described above, the reagent vessels and the amplification vessels are optionally overlaid with a layer of mineral oil to prevent or reduce the evaporation of the solutions in the vessels, which may increase the concentration of the signal antibody conjugate or the amplification conjugate. In general, the solutions in the vessels have a volume of about 50-300 µL, preferably 100-200 µL. The mineral oil layer typically has a volume of 20-80 µL, or 30-50 µL. Mineral oil is commonly used to minimize evaporation and subsequent condensation in PCR sample tubes. The inventor has demonstrated that the probe and immune complexes at the probe tip are not affected by the passage through the mineral oil layer.

### Probe Comprising an Immobilized Hapten

The present invention utilizes a probe comprising a hapten immobilized on the tip of the probe, wherein the probe has an aspect ratio of length to width of at least 5 to 1, the diameter of the probe tip surface is ≤ 5 mm, and the hapten does not substantially dissociate from the probe after an acidic treatment; i.e., no more than 15%, preferably no more than 10% or 5% of the hapten is dissociated from the probe after 1-20 cycles of the acid treatment. The acid treatment is typically performed by dipping the probe in a low pH buffer (pH 1-4, or 1-3, or 1.5-2.5) for 10 seconds to 2 minutes.

The invention is illustrated further by the following examples that are not to be construed as limiting the invention in scope to the specific procedures described in them.

### EXAMPLES

### Example 1: Hapten and Fluorescent Dye Labeling

Fluorescein (F) was labeled to BSA by a standard method. Biotin (B) was linked by a standard method to anti-procalcitonin (PCT) and anti-troponin I (TnI), both from Hytest Ltd. Digoxigenin (Dig)NHS (Sigma Aldrich) was reacted with BSA at a molar coupling ratio (MCR) of 10 for 1 hour at room temperature (RT) followed by purification on a Sephadex G25 (GE Healthcare) column. Cy5^{tm}NHS (GE Healthcare) was reacted with either anti-PCT antibody, anti-TnI antibody, anti B antibody (Jackson Immunoresearch) or streptavidin (Prozyme) at a MCR of 10 in PBS pH 7.4 for 1 hour at RT followed by G25 column purification. AlexaFluor^{™}647 NHS (Thermo Fisher) was labeled to anti-PCT at a MCR of 10 in PBS pH 7.4 for 1 hour at RT followed an G-24 column purification.

### Example 2A: Antibody-Antibody conjugation

Linkage of anti F (Jackson Immunoresearch) to anti-PCT followed established SMCC (succinimidyl 4-[N-malemidomethyl]cyclohexan-1-carboxylate) conjugation chemistry(Hermanson, G.T. (2008) Bioconjugate Techniques, 2nd Ed. Academic Press, New York. ). SMCC (Sigma Aldrich) reacted with anti F at a MCR 0f 15 in PBS Ph 7.4. After 1 hour at room temperature, the antibody was purified with a G-25 column. Anti-PCT reacted with succinimydyl 6-[3-[2-pyridyldithio]-proprionamido]hexanoate (SPDP, Sigma Aldrich) at a MCR of 15 for 1 hour at RT in PBS pH 7.4 followed by purification by G-25. Reaction of the anti-PCT-SPDP with dithiotheritol (DTT,Sigma Aldrich) deprotects the sulfhydryl (SH) and after removal of DTT by G-25 chromatography, the anti-PCT-SH is mixed with anti F-SMCC and allowed to react overnite at RT. The resulting anti F-anti-PCT conjugate was purified with a Sepharose 4B CL (GE Healthcare) column. Conjugation of anti F to anti-TnI and anti-Dig (Jackson Immunoresearch) to anti-PCT followed the same procedure.

### Example 2B: Antibody-Antibody conjugation with FICOLL^{®®}

An alternative dual antibody conjugate of anti-hapten and anti-PCT was prepared as follows. 2 mgs of amino FICOLL^{®} (Skold Technology) in PBS, pH 7.4, was reacted with SPDP (ThermoFisher) at a molar coupling ratio of 10 to 1 for 1 hour followed by an overnight dialysis. De-protection of the thiols occurred by adding 30 µl of DTT (ThermoFisher). After 1 hour, the material was purified on a PD10 column.

2 mgs of anti-hapten, either anti-fluorescein (Jackson Immunoresearch) or anti digoxigennin (Thermo Fisher), were mixed with 2 mgs anti-PCT (Hytest) and reacted with SMCC (ThermoFisher) at a molar coupling ratio of 15 to 1 for 1 hour followed by purification on a PD10 column.

The SMMC labeled antibody mixture was reacted with the thiolated FICOLL^{®} overnight, then it was purified on a Sepharose CL-6B column (GE Healthcare), to prepare FICOLL^{®} linked with anti-fluorescein and anti-PCT. This method was also used to prepare other dual antibody conjugates such as anti-fluorescein and anti-TnI.

### Example 3: Crosslinked FICOLL^{®} Preparation

Preparation of crosslinked FICOLL^{®}, Cy5-anti F-crosslinked FICOLL^{®}, and Cy5-streptavidin-crosslinked FICOLL^{®} followed procedures described U.S. Patent No. 8,492,139. 2 ml of FICOLL^{®} 400 (Sigma/Aldrich) that was aminated to contain 88 amines per FICOLL^{®} 400 kD (Skold Technology) at 20 mg/ml in PBS was added 10 µL of SPDP (at 50 mg/ml in DMF (N,N-Dimethylformamide). The SPDP to FICOLL^{®} MCR was 15. The mixture reacted for 1 hour at room temperature and followed by dialysis. Thiol incorporation was estimated to be 5.5 per FICOLL^{®} 400 kD by standard methods.

To deprotect the thiols on SPDP-labeled FICOLL^{®} 400, 30 µL of DTT at 38 mg/ml PBS was added to 20 mg in 1 ml PBS and allowed to react for two hours at room temperature. The SH-FICOLL^{®} was purified on a PD10 column.

SMCC was linked to aminated FICOLL^{®} 400 (88 amines/FICOLL^{®}) as follows: Aminated FICOLL^{®} 400 at 10 mg in 1 ml PBS was mixed with 25 µL SMCC (Pierce Chemical) at 10 mg/ml DMF for a SMCC/FICOLL^{®} MCR of 30. The mixture reacted for 1 hour at room temperature and followed by purification on a PD10 column (GE Healthcare).

To crosslink the SH-FICOLL^{®} 400 and SMCC-FICOLL^{®} 400, 10 mg in 1ml PBS SH-FICOLL^{®} 400 was mixed with 10 mg in 1ml PBS SMCC-FICOLL^{®} 400. The mixture reacted for overnight at RT.

To provide linking sites for subsequent antibody or streptavidin conjugation to the crosslinked FICOLL^{®} 400, the residual amines were then reacted with an excess of SPDP. 20 mg of crosslinked FICOLL^{®} 400 was mixed with 75 µL SPDP at 50 mg/ml DMF. The mixture reacted for 1 hour at room temperature followed by dialysis versus PBS.

The SPDP labeled crosslinked FICOLL^{®} 400 preparations were then purified on a Sepharose 4B CL column.

### Example 4: Preparation of Cy5-Anti Biotin-Crosslinked FICOLL^{®}

Cy5-anti B at 1.5 mg/ml in 1 ml PBS was mixed with 1.9 µL SMCC at 5 mg/ml DMF and reacted for 1 hour at room temperature followed by purification on a PD 10 column.

The thiols on crosslinked FICOLL^{®} 400-SPDP were deprotected by adding 30 µL DTT at 38 mg/ml to 0.7 mg crosslinked FICOLL^{®} 400-SPDP in 1 ml PBS and reacting for 1 hour at room temperature followed by a PD 10 column to purify the crosslinked FICOLL^{®}.

The Cy5-anti B-SMCC was mixed with crosslinked FICOLL^{®} 400-SH and reacted overnight at room temperature. 10 µL NEM (N-ethyl-maleimide, Aldrich) at 12.5 mg/ml was then added and reacted for ½ hour at room temperature. The conjugate was then purified on a Sepharose 4B CL column.

### Example 5: Preparation of Cy5-streptavidin-crosslinked FICOLL^{®}

Cy5-streptavidin (SA) at 1.5 mg/ml in 1 ml PBS was mixed with 5 µL SMCC at 5 mg/ml DMF and reacted for 1 hour at room temperature followed by purification on a PD 10 column.

The thiols on crosslinked FICOLL^{®} 400-SPDP were deprotected by adding 30 µL DTT at 38 mg/ml to 0.7 mg crosslinked FICOLL^{®} 400-SPDP in 1 ml PBS and reacting for 1 hour at room temperature followed by a PD 10 column to purify the crosslinked FICOLL^{®}.

The Cy5-SA-SMCC was mixed with crosslinked FICOLL^{®} 400-SH and reacted overnight at room temperature. 10 µL NEM (N-ethyl-maleimide, Aldrich) at 12.5 mg/ml was then added and reacted for ½ hour at room temperature. The conjugate was then purified on a Sepharose 4B CL column.

### Example 6: Preparation of Hapten Coated Probes

Quartz probes, 1 mm diameter and 2 cm in length, were coated with aminopropylsilane using a chemical vapor deposition process (Yield Engineering Systems, 1224P) following manufacturer's protocols. The probe tip was then immersed in a solution of either F-bovine serume albumin (BSA) or Dig-BSA, 30 µg/ml in PBS at pH 7.4. After allowing the BSA to adsorb to the probe for 10 minutes, the probe tip was washed in PBS.

### Example 7: Recycle Immunoassays

Reagents and samples were assayed at 120 µL using PHS pH 7.4, 0.05% Tween 20, 5 mg/ml as buffer with 40 µL mineral oil added. Wash buffer was PBS pH 7.4, 0.05% Tween 20 at 200 µL. The probe was held stationary with the microwells stationed on an orbital shaker platform having a 1mm diameter stroke. Orbital flow was used to accelerate binding kinetics. Protocols A, B, C and D describe details of assay binding steps, timing, flow rates and reagent concentrations. Elution reagents were 10 mM glycine/HCl, 200 µL; anhydrous DMSO (Sigma Aldrich) 200 µL; and anhydrous DMSO plus 0.5 mg/ml sodium borohydride (Sigma Aldrich).

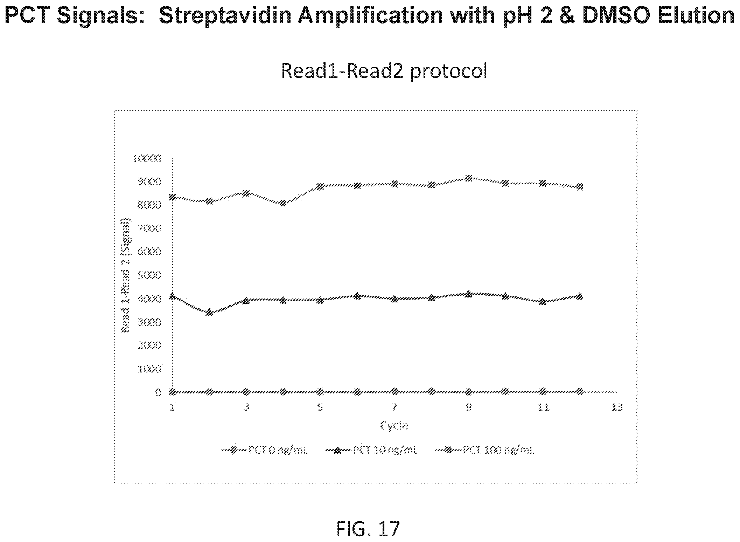

Probe recycle assay formats and assay data are presented in FIG.s 1-16. FIG. 17 illustrates the optical configuration, for detection of fluorescence on the probe tip. Subtracting the Pre-read from the Read generates the immuno-specific signals.

### Example 8: Assay Protocols

The dual antibody conjugate of Protocol A, v1 (Table 1) comprises anti-hapten antibody and capture antibody linked together without a spacer.

The dual antibody conjugate of all other protocols in this Example (Tables 2-11) comprise anti-hapten antibody and capture antibody linked together through a cross-linked FICOLL^{®} 400 spacer.

Wash= PBS with Tween-20

**Table 1**

| **Protocol A, v1** | | | |
|---|---|---|---|
| | **Probe: BSA-F** | | |
| **Step** | **time, sec.** | **rpm** | |
| 1 | 10 | 0 | Pre-read |
| 2 | 180 | 1200 | anti F-anti-PCT @ 30 µg/ml |
| 3 | 30 | 1200 | Wash |
| 4 | 180 | 1200 | PCT sample |
| 5 | 30 | 1200 | Wash |
| 6 | 60 | 1200 | Cy 5-anti-PCT @ 10 µg/ml |
| 7 | 30 | 1200 | Wash |
| 8 | 10 | 0 | Read |
| 9 | 30 | 1200 | pH 2 elution |
| 10 | 10 | 1200 | Wash |
| 11 | Return to 1 | | |

**Table 2**

| **Protocol A, v2** | | | |
|---|---|---|---|
| | **Probe: BSA-F** | | |
| **Step** | **time, sec.** | **rpm** | |
| 1 | 10 | 0 | Pre-read |
| 2 | 180 | 1200 | anti F-anti-TnI @ 30 µg/ml |
| 3 | 30 | 1200 | Wash |
| 4 | 180 | 1200 | Tni sample |
| 5 | 30 | 1200 | Wash |
| 6 | 60 | 1200 | Cy 5-anti-TnI @ 10 µg/ml |
| 7 | 30 | 1200 | Wash |
| 8 | 10 | 0 | Read |
| 9 | 30 | 1200 | pH 2 elution |
| 10 | 10 | 1200 | Wash |
| 11 | Return to 1 | | |

**Table 3**

| **Protocol A, v3** | | | |
|---|---|---|---|
| | **Probe: BSA-F** | | |
| **Step** | **time, sec.** | **rpm** | |
| 1 | 10 | 0 | Pre-read |
| 2 | 180 | 1200 | anti F-anti-PCT @ 30 µg/ml |
| 3 | 30 | 1200 | Wash |
| 4 | 180 | 1200 | PCT sample |
| 5 | 30 | 1200 | Wash |
| 6 | 60 | 1200 | AF-anti-PCT @ 10 µg/ml |
| 7 | 30 | 1200 | Wash |
| 8 | 10 | 0 | Read |
| 9 | 30 | 1200 | pH 2 elution |
| 10 | 10 | 1200 | Wash |
| 11 | Return to 1 | | |

**Table 4**

| **Protocol A, v4** | | | |
|---|---|---|---|
| | **Probe: BSA-Dig** | | |
| **Step** | **time, sec.** | **rpm** | |
| 1 | 10 | 0 | Pre-read |
| 2 | 180 | 1200 | anti Dig-anti-PCT @ 30 µg/ml |
| 3 | 30 | 1200 | Wash |
| 4 | 180 | 1200 | PCT sample |
| 5 | 30 | 1200 | Wash |
| 6 | 60 | 1200 | Cy-anti-PCT @ 10 µg/ml |
| 7 | 30 | 1200 | Wash |
| 8 | 10 | 0 | Read |
| 9 | 30 | 1200 | pH2 elution |
| 10 | 10 | 1200 | Wash |
| 11 | Return to 1 | | |

**Table 5**

| **Protocol B, v1** | | | |
|---|---|---|---|
| | **Probe: BSA-F** | | |
| **Step** | **time, sec.** | **rpm** | |
| 1 | 10 | 0 | Pre-read |
| 2 | 180 | 1200 | anti F-anti-PCT @ 30 µg/ml |
| 3 | 30 | 1200 | Wash |
| 4 | 180 | 1200 | PCT sample |
| 5 | 30 | 1200 | Wash |
| 6 | 60 | 1200 | B-anti-PCT @ 5 µg/ml |
| 7 | 30 | 1200 | Wash |
| 8 | 60 | 1200 | Cy5-anti B-FICOLL^{®} @ 10 µg/mL |
| 9 | 30 | 1200 | Wash |
| 10 | 10 | 0 | Read |
| 11 | 30 | 1200 | pH 2 elution |
| 12 | 10 | 1200 | Wash |
| 13 | Return to 1 | | |

**Table 6**

| **Protocol B, v2** | | | |
|---|---|---|---|
| | **Probe: BSA-F** | | |
| **Step** | **time, sec.** | **rpm** | |
| 1 | 10 | 0 | Pre-read |
| 2 | 180 | 1200 | anti F-anti-PCT @ 30 µg/ml |
| 3 | 30 | 1200 | Wash |
| 4 | 180 | 1200 | PCT sample |
| 5 | 30 | 1200 | Wash |
| 6 | 60 | 1200 | B-anti-PCT @ 5 µg/ml |
| 7 | 30 | 1200 | Wash |
| 8 | 60 | 1200 | Cy5-SA-FICOLL^{®} @ 10 µg/ml |
| 9 | 30 | 1200 | Wash |
| 10 | 10 | 0 | Read |
| 11 | 30 | 1200 | pH 2 elution |
| 12 | 10 | 1200 | Wash |
| 13 | Return to 1 | | |

**Table 7**

| **Protocol C** | | | |
|---|---|---|---|
| | **Probe: BSA-F** | | |
| **Step** | **time, sec.** | **rpm** | |
| 1 | 10 | 0 | Pre-read |
| 2 | 180 | 1200 | anti F-anti-PCT @ 30 µg/ml |
| 3 | 30 | 1200 | Wash |
| 4 | 180 | 1200 | PCT sample |
| 5 | 30 | 1200 | Wash |
| 6 | 60 | 1200 | B-anti-PCT @ 5 µg/ml |
| 7 | 30 | 1200 | Wash |
| 8 | 60 | 1200 | Cy5-SA-FICOLL^{®} @ 10 µg/ml |
| 9 | 30 | 1200 | Wash |
| 10 | 10 | 0 | Read |
| 11 | 60 | 1200 | pH 2 elution |
| 12 | 30 | 1200 | Wash |
| 13 | 60 | 1200 | DMSO Elution |
| 14 | 10 | 1200 | Wash |
| 15 | Return to 1 | | |

**Table 8**

| **Protocol D (not covered by the claimed invention)** | | | |
|---|---|---|---|
| | **Probe: BSA-F** | | |
| **Step** | **time, sec.** | **rpm** | |
| 1 | 10 | 0 | Pre-read |
| 2 | 180 | 1200 | anti F-anti-PCT @ 30 µg/ml |
| 3 | 30 | 1200 | Wash |
| 4 | 180 | 1200 | PCT sample |
| 5 | 30 | 1200 | Wash |
| 6 | 60 | 1200 | B-anti-PCT @ 5 µg/ml |
| 7 | 30 | 1200 | Wash |
| 8 | 60 | 1200 | Cy5-SA-FICOLL^{®} @ 10 µg/ml |
| 9 | 30 | 1200 | Wash |
| 10 | 10 | 0 | Read 1 |
| 11 | 60 | 1200 | pH 2 elution |
| 12 | 0 | 0 | Read 2 |
| 13 | 30 | 1200 | Wash |
| 14 | 60 | 1200 | DMSO Elution |
| 15 | 40 | 1200 | Wash |
| 16 | Return to 1 | | |

**Table 9**

| **Protocol D-1** | | | |
|---|---|---|---|
| | **Probe: BSA-F** | | |
| **Step** | **time, sec.** | **rpm** | |
| 1 | 0 | 0 | Pre-read |
| 2 | 180 | 1200 | anti F-anti PCT |
| 3 | 30 | 1200 | PBST wash |
| 4 | 180 | 1200 | PCT sample |
| 5 | 30 | 1200 | PBST wash |
| 6 | 60 | 1200 | B-anti-PCT |
| 7 | 30 | 1200 | PBST wash |
| 8 | 30 | 1200 | Cy5-SA-cxFicoll |
| 9 | 30 | 1200 | PBST wash |
| 10 | 0 | 0 | Read 1 |
| 11 | 30 | 1200 | Guanidine HCl, pH 1.6 |
| 12 | 0 | 0 | Read 2 |
| 13 | 30 | 1200 | PBST wash |
| 14 | 60 | 1200 | DMSO |
| 15 | 30 | 1200 | PBST wash |
| 16 | Return to 1 | | |

**Table 10**

| **Protocol E (not covered by the claimed invention)** | | | |
|---|---|---|---|
| | **Probe: BSA-F** | | |
| **Step** | **time, sec.** | **rpm** | |
| 1 | 600 | 0 | Anti F-anti-PCT+PCT+biotin-anti-PCT |
| 2 | | | Load F-BSA probe and start assay |
| 3 | 0 | 0 | Pre-read |
| 4 | 300 | 1200 | anti F-anti-PCT+PCT+biotin-anti-PCT (from step 1) |
| 5 | 30 | 1200 | Wash |
| 6 | 30 | 1200 | Cy5-SA-FICOLL^{®} @ 10 µg/ml |
| 7 | 30 | 1200 | Wash |
| 8 | 0 | 0 | Read 1 |
| 9 | 30 | 1200 | pH 2 elution |
| 10 | 0 | 0 | Read 2 |
| 11 | 30 | 1200 | Wash |
| 12 | 60 | 1200 | DMSO Elution |
| 13 | 30 | 1200 | Wash |
| 14 | Return to 1 | | |

**Table 11**

| **Protocol F** | | | |
|---|---|---|---|
| | **Probe: BSA-F** | | |
| **Step** | **time, sec.** | **rpm** | |
| 1 | 0 | 0 | Pre-read |
| 2 | 180 | 1200 | anti F-anti SAA |
| 3 | 30 | 1200 | Three wash |
| 4 | 180 | 1200 | SAA sample |
| 5 | 30 | 1200 | Three wash |
| 6 | 60 | 1200 | Cy5-anti-SAA |
| 7 | 30 | 1200 | Three wash |
| 8 | 0 | 0 | Read 1 |
| 9 | 30 | 1200 | Guanidine HCL pH 1.6 |
| 10 | 0 | 0 | Read 2 |
| 11 | 30 | 1200 | PBST |
| 12 | 60 | 1200 | DMSO |
| 13 | 30 | 1200 | PBST |
| 14 | Return to 1 | | |

### Example 9. PCT Assay Result (Protocol A, v1)

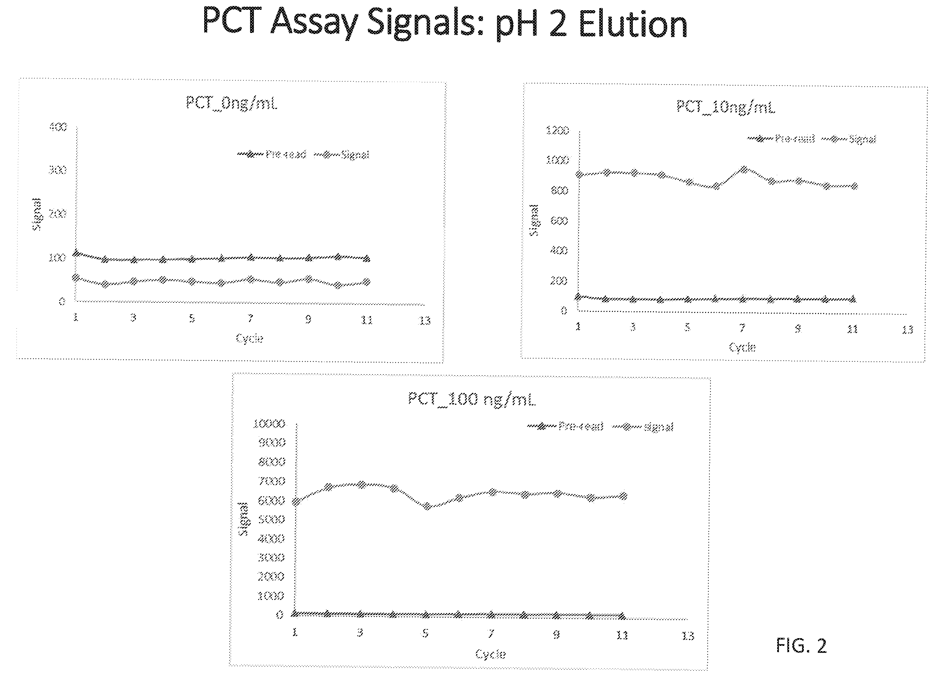

FIG. 2 shows data of PCT assays following protocol A, v1 with the fluorescein probe format described in FIG. 1B. PCT samples from negative, 10 ng/ml and 100 ng/ml show consistent fluorescence signals thru 10 cycles. The 100 ng/ml samples represented the maximum signal in this PCT assay. Previous experiments showed steady increase in signal upon each cycle, which was attributed to evaporation in the capture AB and signal AB reagents. Since the reagent volumes were only 120 µL, aqueous evaporation likely caused an increase in AB concentration. By adding 40 µL of mineral oil forming a layer on top of the reagents to minimize aqueous evaporation, consistent signals were achieved as in FIG. 2. It is remarkable that the probe tip containing immune complexes were not adversely affected by transit of the probe tip thru the mineral oil layer.

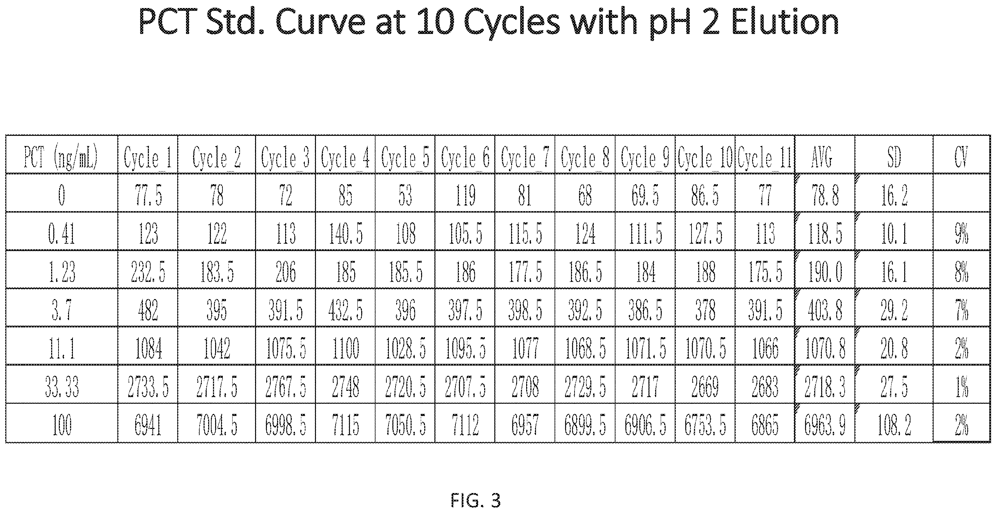

FIG. 3 shows the data of PCT standards in the fluorescein coated probe format (FIG. 1B) with protocol A, v1. Results at each PCT level are very consistent across 10 cycles, CV <10%. The high reproducibility of the assay indicates the method can be applied to quantitative applications and only a single calibration curve is necessary, rather than cycle specific calibration.

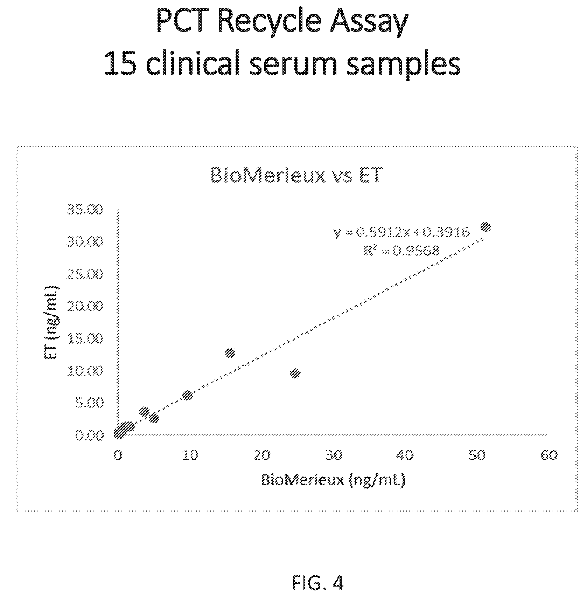

FIG. 4 shows that the results with PCT clinical serum samples having correlation (R² = 0.95) of the recycle assay with a commercial IVD instrument (Biomerieux *Vidas).* To measure 15 samples, the *Vidas* used 15 test strips, while the recycle assay used a single probe and reagent set with samples measured in random order. The recycle assay format can tolerate biological samples and accurate results are obtainable.

### Example 10. TnI Assay Result (Protocol A, v2)

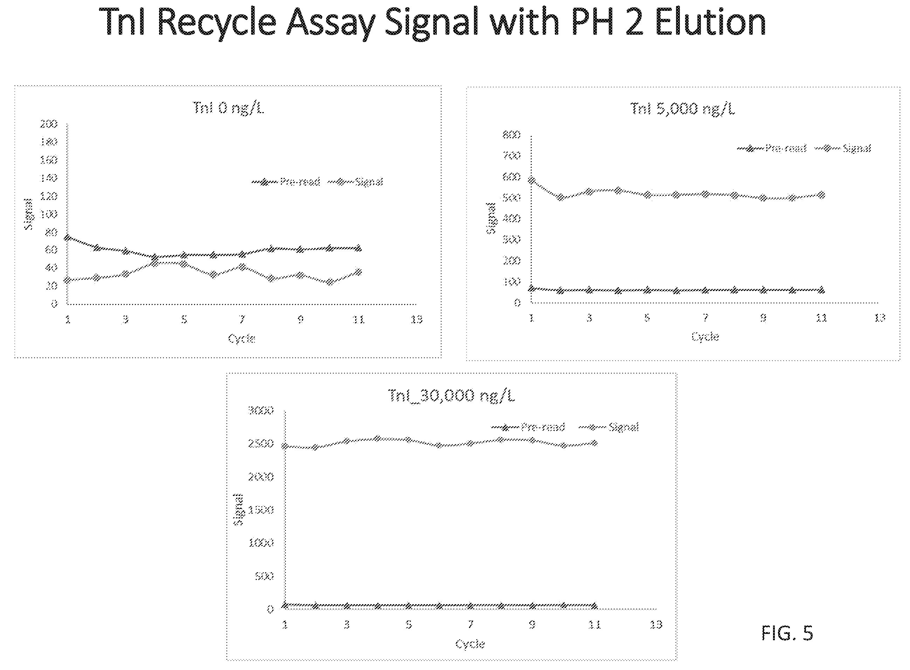

FIG. 5 presents results applying the recycle assay (Protocol A, v2) to another immunoassay, cardiac TnI. The format is as FIG. 1B, except capture AB was anti F linked to anti-TnI and signal AB was Cy5 -anti-TnI.

### Example 11. PCT Assay Result (Protocol A, v3)

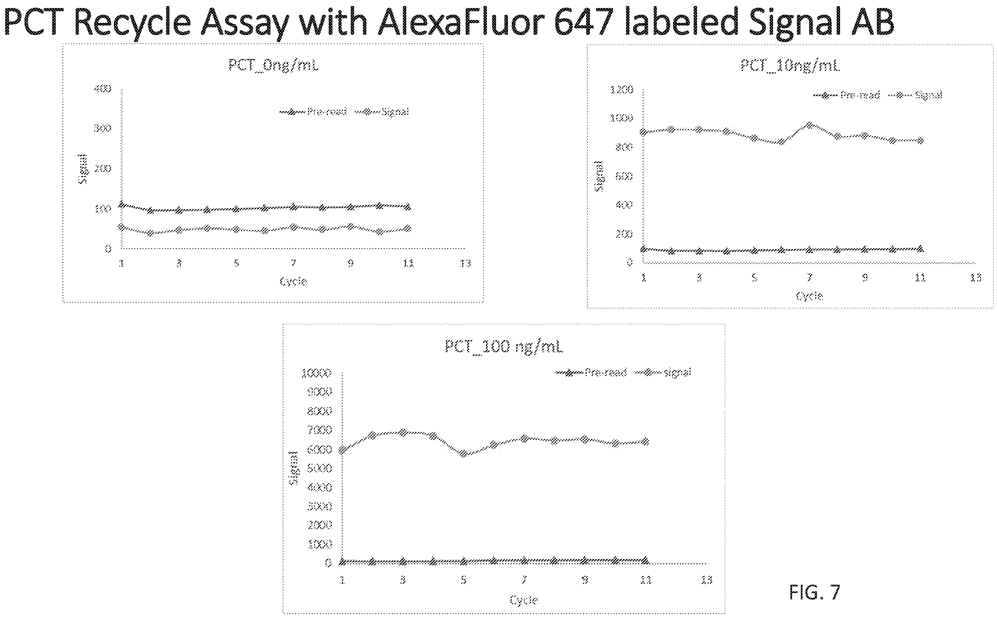

FIG. 7 contains data of different PCT samples up 10 cycles using the Alexa Fluor 647 labeled signal AB (Protocol A, v3). Results show that the invention can be applied to a multitude of signal generating dyes (fluorescent and chemiluminescent). The principle of the invention centers upon dissociation of an anti hapten antibody from a hapten coated probe surface. The dye employed should not impact the hapten/ anti hapten disassociation since the dye is labeled to the signal AB.

### Example 12. PCT Assay Result (Protocol A, v4)

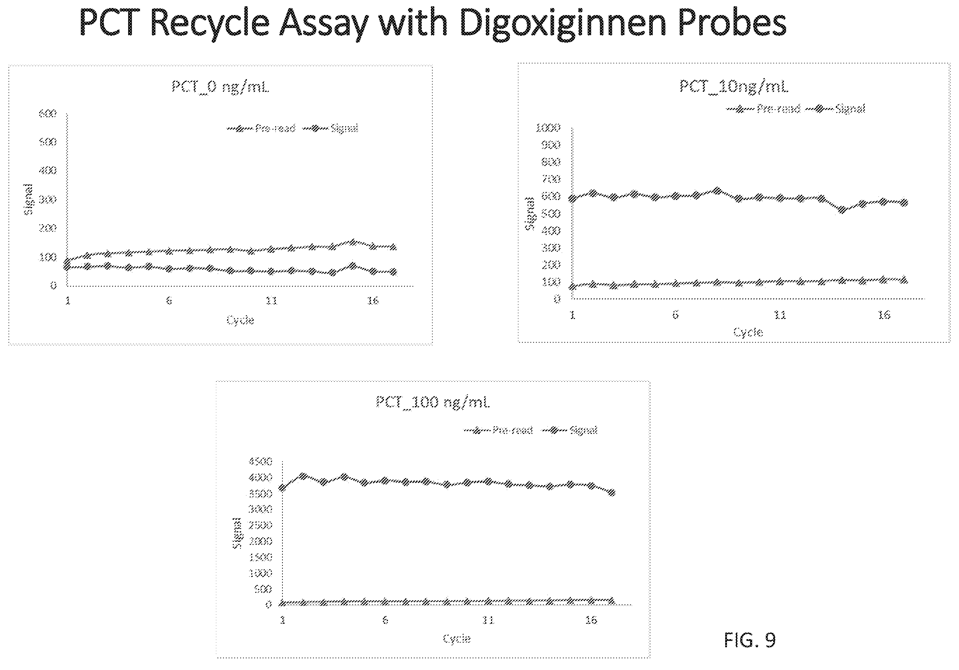

FIG. 9 presents PCT levels up to 10 cycles using the digoxiginnen format (Protocol A,v4) results are very similar to the fluorescein probe format in FIG. 3. Results indicate the invention can employ multiple hapten / anti hapten pairs in the probe coating.

### Example 13. PCT Assay Result (Protocol B)

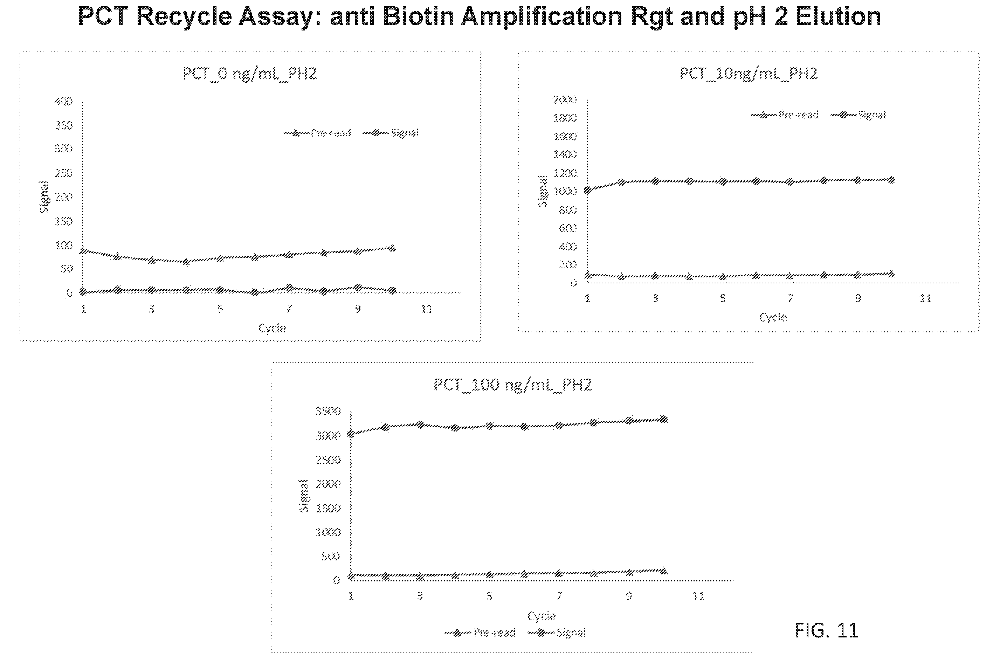

FIG. 11 shows PCT assays performed with the recycle format in FIG. 10 with Protocol B, v1 using pH 2 elution. FIG.s 11 shows 3 PCT levels up to 10 cycles demonstrating feasibility of using a labeled antibody linked to a high molecular weight polymer.

### Example 14. PCT Assay Result (Protocol B, v2)

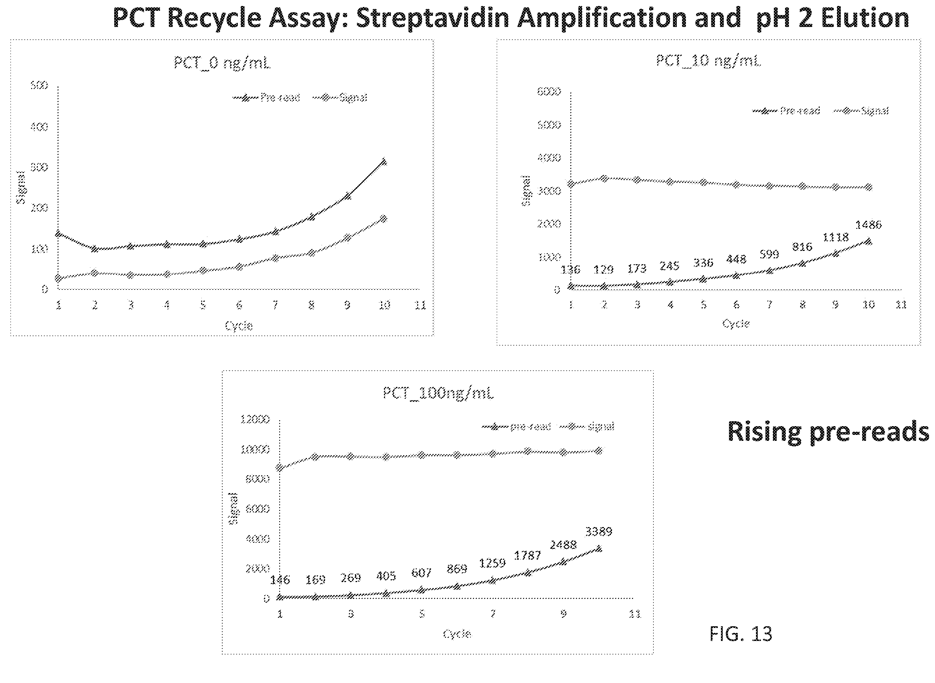

FIG. 13 shows the PCT results with the streptavidin amplification reagent using Protocol B, v2 with pH 2 elution. The data show raising pre-read at each cycle. Subtracting the pre-read signal from the final read signal is used to derive the PCT specific signal. In this case, the PCT specific signals remain constant thru 10 cycles. The data suggests that besides PCT immune complexes additional fluorescent dye is bound on the probe surface. The likely culprit was believed to be streptavidin since it is known to be robust where even pH 2 treatment would not inhibit its biotin binding activity. Any residual streptavidin remaining on the probe tip after the pH 2 elution could remain active in subsequent cycles.

### Example 15. PCT Assay Result (Protocol C)

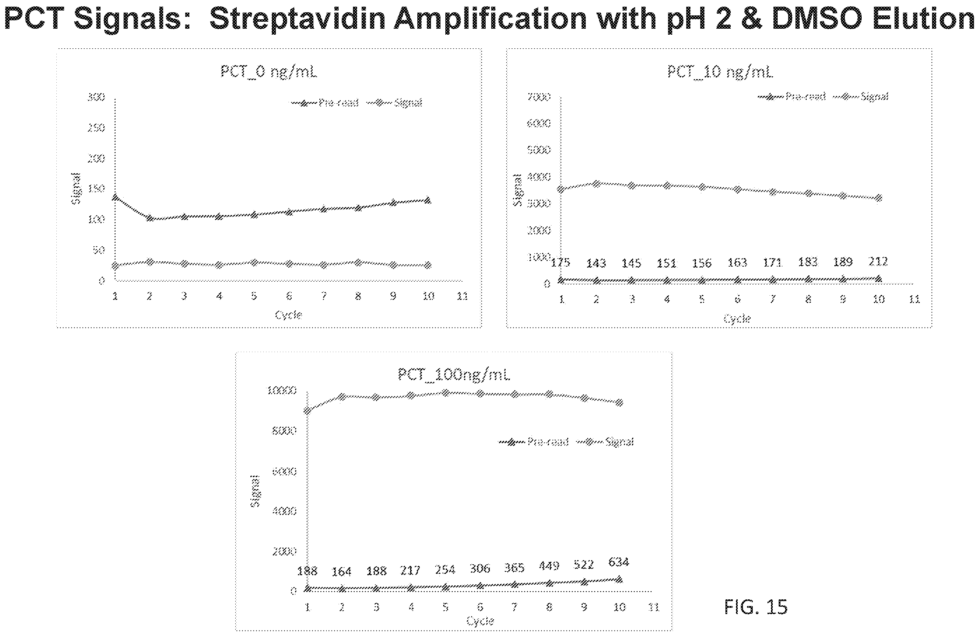

FIG. 15 contains the results of the recycle assay of 3 PCT levels with the dual pH 2 and DMSO elution as in FIG. 14 using Protocol C. PCT signals remain consistent through 10 cycles with a significant reduction in pre-read signals. Although many organic solvents could serve as suitable denaturants, DMSO would be one of the preferred choices due to its relatively low toxicity. Consistent PCT cycles also indicates that the hapten coated probe surface tolerates the repeated DMSO exposures.

### Example 16A. PCT Assay Result (Protocol D; not covered by the claimed invention)

FIG. 17 shows the results of the recycle assay of 3 PCT levels (0, 10, and 100 ng/mL) by the two-read/ subtraction protocol (FIG. 16B, Protocol D). In 12 cycles, the fluorescent signals of PCT = 0 ng/mL remain negligible (about 20-30 fluorescent units), which is significantly lower than the results shown in FIG. 15 (between 80-110 fluorescent units after subtraction of pre-read fluorescent signal).

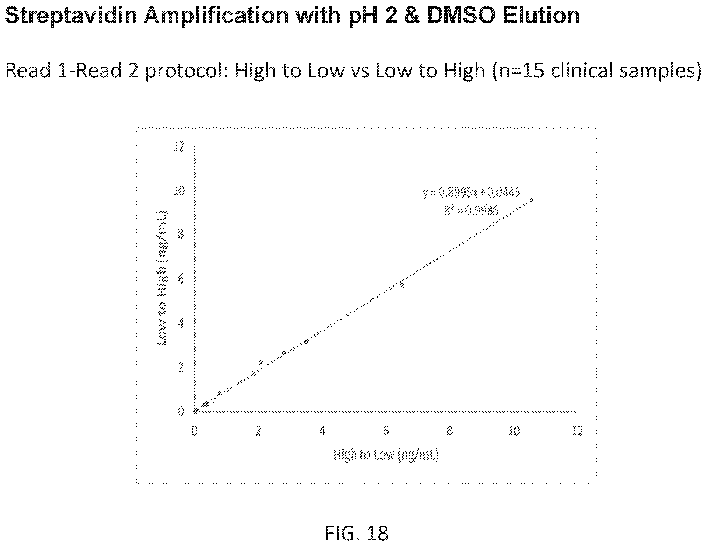

FIG. 18 shows that the results with 15 PCT clinical serum samples by two-read recycle protocol (Protocol D). The 15 samples were measured twice in the order from low concentration to high concentration (shown as Y-axis) and from high concentration to low concentration (shown as X-axis). The results of high to low and low to high are highly correlated (R² = 0.9985) indicating no carry-over problem regardless the sample concentration.

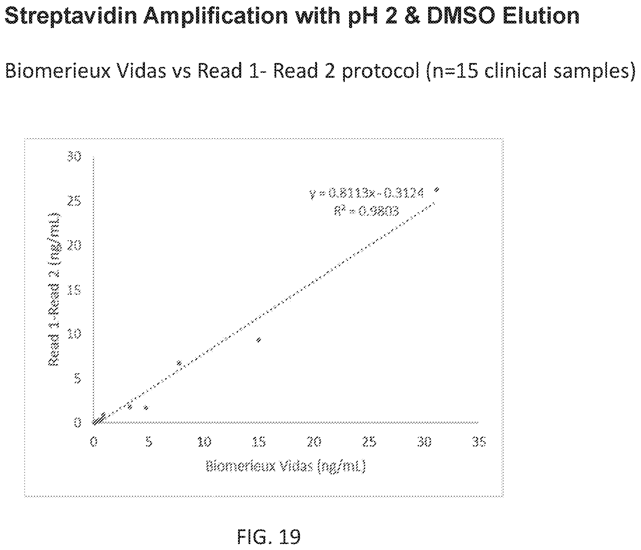

FIG. 19 shows that the results with 15 PCT clinical serum samples had high correlation (R² = 0.98) of two-read recycle protocol (Protocol D) vs. a commercial IVD instrument (Biomerieux *Vidas).* To measure 15 samples, the *Vidas* used 15 test strips, while the recycle assay used a single probe and reagent set with samples measured in random order.

### Example 16B. PCT Assay Result (Protocol D-1)

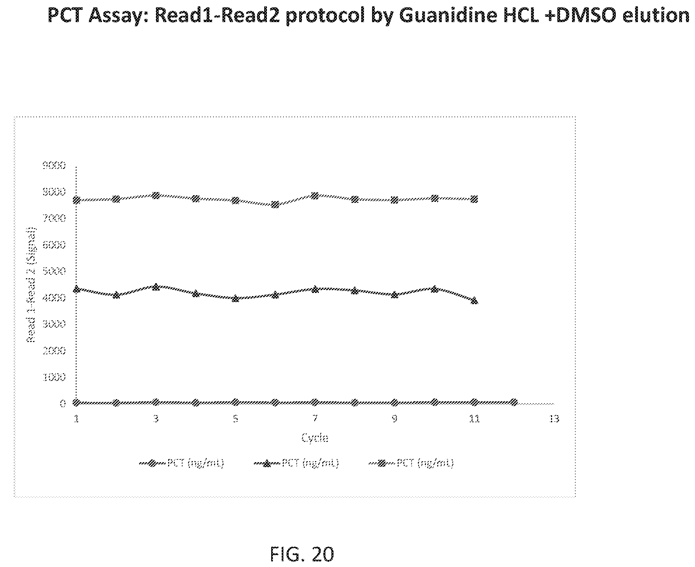

FIG. 20 contains the results of the recycle assay of 3 PCT levels with the dual low pH plus denaturant guanidine chloride (6M guanidine.HCl, pH 1.6) and DMSO elution using Protocol D-1. The protocol is similar to those described in FIG. 16B, except the first elution was carried out in 6M guanidine hydrochloride (pH 1.6). Similar to Protocol D, PCT signals remain consistent through 11 cycles with a significant reduction in pre-read signals. Consistent PCT cycles also indicates that the hapten coated probe surface tolerates the repeated denaturants guanidine chloride and DMSO exposures.

### Example 17. PCT Assay Result (Protocol E; not covered by the claimed invention)

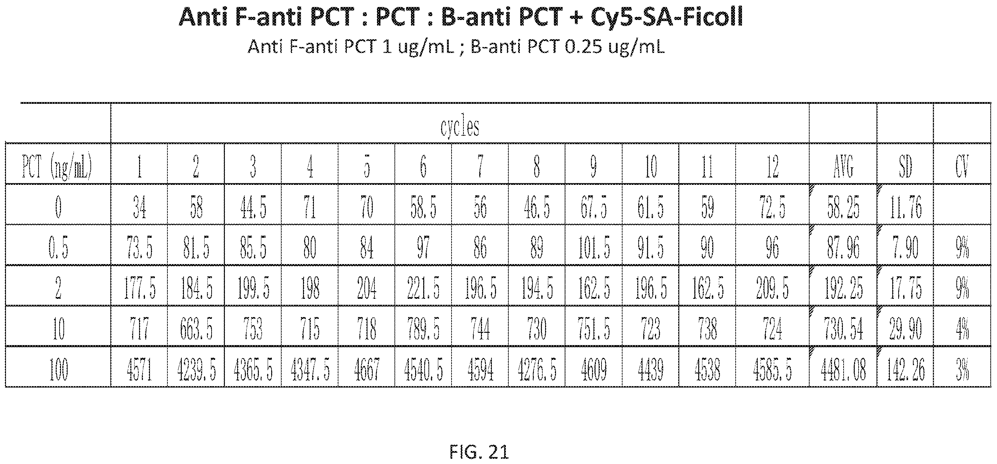

FIG. 21 shows the data of PCT standards in the fluorescein-coated probe format (FIG. 16A) with protocol E. Sample was mixed with dual antibody anti-F-anti-PCT and signal antibody biotin-anti-PCT first before contacting the probe. Results at each PCT level are very consistent across 12 cycles, CV <10%.

### Example 18. Serum Amyloid A (SAA) Assay Result (Protocol F)

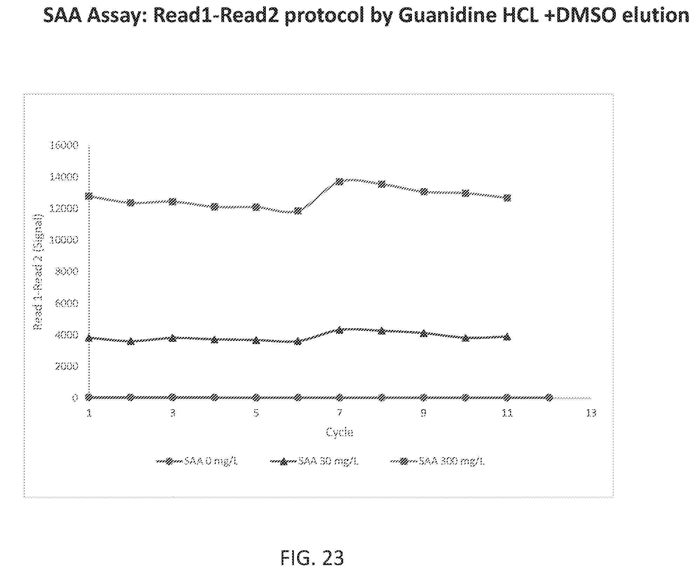

FIG. 23 contains the results of the recycle assay of 3 SAA levels with the dual (i) low pH plus denaturant guanidium chloride (6M guanidine.HCl, pH 1.6) elution and (ii) denaturant DMSO elution as in FIG. 22 using Protocol F. Recombinant SAA, capture antibody monoclonal SAA antibody (6F9) and signal antibody monoclonal SAA antibody (8C7) were obtained from GenScript.

SAA tends to aggregate and sticks to probe. Without guanidium chloride, the pH 2 acid elution and the DMSO elution did not appear to disassociate the immune complex substantially from the probe. The pre-read signal got progressively higher each cycle causing the specific SAA binding signal declined upon each cycle (data not shown here).

However, the inventors discovered that by adding guanidium chloride in the acid elution, i.e., with both (i) low pH plus denaturant guanidium chloride elution and (ii) denaturant DMSO elution, the pre-read signals remain low and SAA signals remain consistent through 11 cycles (see FIG. 23).

The invention, and the manner and process of making and using it, are now described in such full, clear, concise and exact terms as to enable any person skilled in the art to which it pertains, to make and use the same. It is to be understood that the foregoing describes preferred embodiments of the present invention and that modifications may be made therein without departing from the scope of the present invention as set forth in the claims. To particularly point out and distinctly claim the subject matter regarded as invention, the following claims conclude this specification.

## Claims

1. A method of detecting an analyte in multiple liquid samples comprising an analyte, comprising the steps of:
(a) dipping a probe in an aqueous solution having pH of 6.0-8.5 to pre-read the fluorescent signal of the probe tip, wherein the probe comprises a hapten immobilized on the tip of the probe, and the diameter of the tip surface is ≤ 5 mm;
(b) forming an immunocomplex comprising the analyte from a sample, a capture antibody, and a signal antibody on the probe tip, wherein the capture antibody and the signal antibody are two different antibodies against the analyte, the capture antibody is covalently linked to an anti-hapten antibody against the hapten and the signal antibody is conjugated with fluorescent labels;
(c) dipping the probe tip into a washing vessel containing a wash solution;
(d) determining the analyte concentration in the sample by measuring the fluorescent signal of the immunocomplex at the probe tip, subtracting the pre-read fluorescent signal of (a), and quantitating the subtracted signal against a calibration curve;
(e) dipping the probe tip in an acidic solution having pH about 1.0-4.0 to elute the immunocomplex from the probe tip; and
(f) repeating the steps (a)-(e) except in step (b) with the analyte from a new sample, whereby the analyte in each of the multiple liquid samples is detected.

2. The method of Claim 1, wherein the immunocomplex of step (b) is formed by:
(b1) mixing a sample solution with a dual antibody solution and a reagent solution to form a mixture, wherein the sample solution comprises the analyte, the dual antibody solution comprises the anti-hapten antibody covalently linked to the capture antibody, and the reagent solution comprises the signal antibody conjugated with fluorescent labels; and
(b2) dipping the probe tip in the mixture to form the immunocomplex on the probe tip.

3. The method of Claim 1, wherein the immunocomplex of step (b) is formed by:
(b1) dipping the probe tip in a dual antibody solution comprising the anti-hapten antibody covalently linked to the capture antibody;
(b2) dipping the probe tip in a sample solution comprising the sample;
(b3) dipping the probe tip in a reagent solution comprising the signal antibody conjugated with fluorescent labels to form the immunocomplex on the probe tip.

4. The method of Claim 1, 2, or 3, wherein:
i) the acidic solution in step (e) has a pH of 1.5-2.5
ii) the probe tip is exposed to the acidic solution one time for 10 second to 2 minutes, or
iii) the probe tip is exposed to a pulse treatment of 2-5 cycles of the acidic solution treatment followed by neutralization in the read vessel for 10-20 seconds.

5. The method of Claim 1, 2, or 3, wherein the hapten is fluorescein, digoxiginnen, or biotin.

6. A method of detecting an analyte in multiple liquid samples, comprising the steps of:
(a) dipping a probe in an aqueous solution having pH of 6.0-8.5 to pre-read the fluorescent signal of the probe tip, wherein the probe comprises a first hapten immobilized on the tip of the probe, and the diameter of the tip surface is ≤ 5 mm;
(b) forming a first immunocomplex comprising the analyte from a sample, a capture antibody, and a signal antibody on the probe tip, wherein the capture antibody and the signal antibody are two different antibodies against the analyte, the capture antibody is covalently linked to an anti-first hapten antibody against the first hapten and the signal antibody is conjugated with a second hapten, the first hapten and the second hapten are different;
(c) dipping the probe tip in a wash solution;
(d) dipping the probe tip in an amplification solution comprising an anti-second hapten antibody or streptavidin conjugated to fluorescent labels to form a second immunocomplex comprising the analyte, the capture antibody, the signal antibody, the second hapten, and the anti-second hapten antibody or streptavidin on the probe tip;
(e) determining the analyte concentration in the sample by measuring the fluorescent signal of the second immunocomplex at the probe tip, subtracting the pre-read fluorescent signal of (a), and quantitating the subtracted signal against a calibration curve;
(f) dipping the probe tip in an acidic solution having pH about 1.0-4.0 to elute the immunocomplexes from the probe tip; and
(g) repeating the same steps (a)-(f) except in step (b) with the analyte from a new sample, whereby the analyte in each of the multiple liquid samples is detected.

7. The method of Claim 6, wherein the first immunocomplex of step (b) is formed by:
(b1) mixing a sample solution with a dual antibody solution and a reagent solution to form a mixture, wherein the sample solution comprises the analyte, the dual antibody solution comprises the anti-first hapten antibody covalently linked to the capture antibody, and the reagent solution comprises the signal antibody conjugated with the second hapten; and
(b2) dipping the probe tip in the mixture to form the first immunocomplex on the probe tip.

8. The method of Claim 6, wherein the first immunocomplex of step (b) is formed by:
(b1) dipping the probe tip in a dual antibody solution comprising the anti-first hapten antibody covalently linked to the capture antibody;
(b2) dipping the probe tip in a sample solution comprising the sample;
(b3) dipping the probe tip in a reagent solution comprising the signal antibody conjugated with the second hapten to form the first immunocomplex on the probe tip.

9. The method of Claim 6, 7, or 8, wherein the amplification solution comprises an anti-second hapten antibody conjugated to the fluorescent labels.

10. The method of Claim 6, 7, or 8, wherein the first hapten and the second hapten are selected from the group consisting of: fluorescein, digoxiginnen, and biotin, preferably
wherein the first hapten is non-biotin, the second hapten is biotin, and the amplification solution comprises streptavidin conjugated to the fluorescent labels.

11. The method of Claim 6, 7, or 8, wherein the amplification solution comprises copolymers of sucrose and epichlorohydrin bound to the conjugate of the anti-second hapten antibody or streptavidin and the fluorescent labels.

12. A method of detecting an analyte in multiple liquid samples, comprising the steps of:
(a) dipping a probe in an aqueous solution having pH of 6.0-8.5 to pre-read the fluorescent signal of the probe tip, wherein the probe comprises a hapten immobilized on the tip of the probe, and the diameter of the tip surface is ≤ 5 mm;
(b) forming an immunocomplex comprising the analyte from a sample, a capture antibody, and a signal antibody on the probe tip, wherein the capture antibody and the signal antibody are two different antibodies against the analyte, the capture antibody is covalently linked to an anti-hapten antibody against the hapten and the signal antibody is conjugated with fluorescent labels;
(c) dipping the probe tip into a washing vessel containing a wash solution;
(d) reading a first fluorescent signal of the immunocomplex at the probe tip;
(e) dipping the probe tip in an acidic solution having pH about 1.0-4.0 and containing guanidium chloride;
(f) reading a second fluorescent signal of the acid-treated probe tip,
(g) subtracting the second fluorescent signal from the first fluorescent signal, and quantitating the subtracted signal against a calibration curve;
(h) dipping the probe tip in a chaotropic agent different from guanidium chloride; and
(i) repeating the same steps (b)-(h) except in step (b) with a new sample solution comprising a new sample, whereby the analyte in each of the multiple liquid samples is detected.

13. The method of Claim 12, wherein the first immunocomplex of step (b) is formed by:
(b1) mixing a sample solution with a dual antibody solution and a reagent solution to form a mixture, wherein the sample solution comprises the analyte, the dual antibody solution comprises the anti-hapten antibody covalently linked to the capture antibody, and the reagent solution comprises the signal antibody conjugated with fluorescent labels; and
(b2) dipping the probe tip in the mixture to form the first immunocomplex on the probe tip.

14. The method of Claim 12, wherein the immunocomplex of step (b) is formed by:
(b1) dipping the probe tip in a dual antibody solution comprising the anti-hapten antibody covalently linked to the capture antibody;
(b2) dipping the probe tip in a sample solution comprising the sample;
(b3) dipping the probe tip in a reagent solution comprising the signal antibody conjugated with fluorescent labels to form the first immunocomplex on the probe tip.

15. The method of Claim 1, 6, or 12, wherein the capture antibody is covalently linked to the anti-hapten antibody through copolymers of sucrose and epichlorohydrin.

## Patentansprüche

1. Verfahren zum Detektieren eines Analyten in mehreren Flüssigkeitsproben, die einen Analyten umfassen, umfassend die Schritte:
(a) Tauchen einer Sonde in eine wässrige Lösung mit einem pH-Wert von 6,0 bis 8,5 zum vorherigen Ablesen des Fluoreszenzsignals der Sondenspitze, wobei die Sonde ein Hapten umfasst, das auf der Spitze der Sonde immobilisiert ist, und der Durchmesser der Spitzenoberfläche ≤ 5 mm ist;
(b) Bilden eines Immunokomplexes, der den Analyten aus einer Probe, einen Einfang-Antikörper und einen Signal-Antikörper umfasst, auf der Sondenspitze, wobei der Einfang-Antikörper und der Signal-Antikörper zwei unterschiedliche Antikörper gegen den Analyten sind, wobei der Einfang-Antikörper kovalent mit einem Anti-Hapten-Antikörper gegen das Hapten verknüpft ist, und der Signal-Antikörper mit Fluoreszenzmarkierungen konjugiert ist;
(c) Tauchen der Sondenspitze in ein Waschgefäß, welches eine Waschlösung enthält;
(d) Bestimmen der Analytkonzentration in der Probe durch Messen des Fluoreszenzsignals des Immunokomplexes an der Sondenspitze, Subtrahieren des vorher abgelesenen Fluoreszenzsignals von (a), und Quantifizieren des subtrahierten Signals gegen eine Kalibrierkurve;
(e) Tauchen der Sondenspitze in eine saure Lösung mit einem pH-Wert von etwa 1,0 bis 4,0, um den Immunokomplex aus der Sondenspitze zu eluieren; und
(f) Wiederholen der Schritte (a) bis (e) außer in Schritt (b) mit dem Analyten aus einer neuen Probe, wodurch der Analyt in jeder der mehreren Flüssigkeitsproben detektiert wird.

2. Verfahren nach Anspruch 1, wobei der Immunokomplex aus Schritt (b) gebildet wird durch:
(b1) Mischen einer Probenlösung mit einer dualen Antikörperlösung und einer Reagenzlösung, um eine Mischung zu bilden, wobei die Probenlösung den Analyten umfasst, die duale Antikörperlösung den Anti-Hapten-Antikörper umfasst, der kovalent mit dem Einfang-Antikörper verknüpft ist, und die Reagenzlösung den Signal-Antikörper umfasst, der mit Fluoreszenzmarkierungen konjugiert ist; und
(b2) Tauchen der Sondenspitze in die Mischung, um den Immunokomplex auf der Sondenspitze zu bilden.

3. Verfahren nach Anspruch 1, wobei der Immunokomplex aus Schritt (b) gebildet wird durch:
(b1) Tauchen der Sondenspitze in die duale Antikörperlösung, die den Anti-Hapten-Antikörper umfasst, der kovalent mit dem Einfang-Antikörper verknüpft ist;
(b2) Tauchen der Sondenspitze in eine Probenlösung, welche die Probe umfasst;
(b3) Tauchen der Sondenspitze in eine Reagenzlösung, die den Signal-Antikörper umfasst, der mit Fluoreszenzmarkierungen konjugiert ist, um den Immunokomplex auf der Sondenspitze zu bilden.

4. Verfahren nach Anspruch 1, 2 oder 3, wobei:
i) die saure Lösung in Schritt (e) einen pH-Wert von 1,5 bis 2,5 hat,
ii) die Sondenspitze der sauren Lösung ein Mal für 10 Sekunden bis 2 Minuten ausgesetzt wird, oder
iii) die Sondenspitze einer Pulsbehandlung von 2 bis 5 Zyklen der Behandlung mit saurer Lösung, gefolgt von Neutralisation in dem Ablesegefäß für 10 bis 20 Sekunden ausgesetzt wird.

5. Verfahren nach Anspruch 1, 2 oder 3, wobei das Hapten Fluorescein, Digoxiginnen oder Biotin ist.

6. Verfahren zum Detektieren eines Analyten in mehreren Flüssigkeitsproben, umfassend die Schritte:
(a) Tauchen einer Sonde in eine wässrige Lösung mit einem pH-Wert von 6,0 bis 8,5 zum vorherigen Ablesen des Fluoreszenzsignals der Sondenspitze, wobei die Sonde ein erstes Hapten umfasst, das auf der Spitze der Sonde immobilisiert ist, und der Durchmesser der Spitzenoberfläche ≤ 5 mm ist;
(b) Bilden eines ersten Immunokomplexes, der den Analyten aus einer Probe, einen Einfang-Antikörper und einen Signal-Antikörper umfasst, auf der Sondenspitze, wobei der Einfang-Antikörper und der Signal-Antikörper zwei unterschiedliche Antikörper gegen den Analyten sind, wobei der Einfang-Antikörper kovalent mit einem Anti-erstes Hapten-Antikörper gegen das erste Hapten verknüpft ist, und der Signal-Antikörper mit einem zweiten Hapten konjugiert ist, wobei das erste Hapten und das zweite Hapten unterschiedlich sind;
(c) Tauchen der Sondenspitze in eine Waschlösung;
(d) Tauchen der Sondenspitze in eine Amplifizierungslösung, die einen Anti-zweites Hapten-Antikörper oder Streptavidin, konjugiert an Fluoreszenzmarkierungen, umfasst, um einen zweiten Immunokomplex zu bilden, der den Analyten, den Einfang-Antikörper, den Signal-Antikörper, das zweite Hapten und den Anti-zweites Hapten-Antikörper oder Streptavidin auf der Sondenspitze umfasst;
(e) Bestimmen der Analytkonzentration in der Probe durch Messen des Fluoreszenzsignals des zweiten Immunokomplexes an der Sondenspitze, Subtrahieren des vorher abgelesenen Fluoreszenzsignals von (a), und Quantifizieren des subtrahierten Signals gegen eine Kalibrierkurve;
(f) Tauchen der Sondenspitze in eine saure Lösung mit einem pH-Wert von etwa 1,0 bis 4,0, um den Immunokomplexes aus der Sondenspitze zu eluieren; und
(g) Wiederholen der gleichen Schritte (a) bis (f) außer in Schritt (b) mit dem Analyten aus einer neuen Probe, wodurch der Analyt in jeder der mehreren Flüssigkeitsproben detektiert wird.

7. Verfahren nach Anspruch 6, wobei der erste Immunokomplex aus Schritt (b) gebildet wird durch:
(b1) Mischen einer Probenlösung mit einer dualen Antikörperlösung und einer Reagenzlösung, um eine Mischung zu bilden, wobei die Probenlösung den Analyten umfasst, die duale Antikörperlösung den Anti-erstes Hapten-Antikörper umfasst, der kovalent mit dem Einfang-Antikörper verknüpft ist, und die Reagenzlösung den Signal-Antikörper umfasst, der mit dem zweiten Hapten konjugiert ist; und
(b2) Tauchen der Sondenspitze in die Mischung, um den ersten Immunokomplex auf der Sondenspitze zu bilden.

8. Verfahren nach Anspruch 6, wobei der erste Immunokomplex aus Schritt (b) gebildet wird durch:
(b1) Tauchen der Sondenspitze in eine duale Antikörperlösung, die den Anti-erstes Hapten-Antikörper umfasst, der kovalent mit dem Einfang-Antikörper verknüpft ist;
(b2) Tauchen der Sondenspitze in eine Probenlösung, welche die Probe umfasst;
(b3) Tauchen der Sondenspitze in eine Reagenzlösung, die den Signal-Antikörper umfasst, der mit dem zweiten Hapten konjugiert ist, um den ersten Immunokomplex auf der Sondenspitze zu bilden.

9. Verfahren nach Anspruch 6, 7 oder 8, wobei die Amplifizierungslösung einen Anti-zweites Hapten-Antikörper umfasst, der an die Fluoreszenzmarkierungen konjugiert ist.

10. Verfahren nach Anspruch 6, 7 oder 8, wobei das erste Hapten und das zweite Hapten ausgewählt sind aus der Gruppe bestehend aus: Fluorescein, Digoxiginnen und Biotin, wobei vorzugsweise das erste Hapten Nicht-Biotin ist, das zweite Hapten Biotin ist, und die Amplifizierungslösung Streptavidin umfasst, das an die Fluoreszenzmarkierungen konjugiert ist.

11. Verfahren nach Anspruch 6, 7 oder 8, wobei die Amplifizierungslösung Copolymere von Sucrose und Epichlorhydrin umfasst, gebunden an das Konjugat von Anti-zweites-Hapten-Antikörper oder Streptavidin und den Fluoreszenzmarkierungen.

12. Verfahren zum Detektieren eines Analyten in mehreren Flüssigkeitsproben, umfassend die Schritte:
(a) Tauchen einer Sonde in eine wässrige Lösung mit einem pH-Wert von 6,0 bis 8,5 zum vorherigen Ablesen des Fluoreszenzsignals der Sondenspitze, wobei die Sonde ein Hapten umfasst, das auf der Spitze der Sonde immobilisiert ist, und der Durchmesser der Spitzenoberfläche ≤ 5 mm ist;
(b) Bilden eines Immunokomplexes, der den Analyten aus einer Probe, einen Einfang-Antikörper und einen Signal-Antikörper umfasst, auf der Sondenspitze, wobei der Einfang-Antikörper und der Signal-Antikörper zwei unterschiedliche Antikörper gegen den Analyten sind, wobei der Einfang-Antikörper kovalent mit einem Anti-Hapten-Antikörper gegen das Hapten verknüpft ist, und der Signal-Antikörper mit Fluoreszenzmarkierungen konjugiert ist;
(c) Tauchen der Sondenspitze in ein Waschgefäß, welches eine Waschlösung enthält;
(d) Ablesen eines ersten Fluoreszenzsignals des Immunokomplexes an der Sondenspitze;
(e) Tauchen der Sondenspitze in eine saure Lösung mit einem pH-Wert von etwa 1,0 bis 4,0, und die Guanidiumchlorid enthält;
(f) Ablesen eines zweiten Fluoreszenzsignals der säurebehandelten Sondenspitze;
(g) Subtrahieren des zweiten Fluoreszenzsignals von dem ersten Fluoreszenzsignal, und Quantifizieren des subtrahierten Signals gegen eine Kalibrierkurve;
(h) Tauchen der Sondenspitze in ein chaotropes Mittel, das sich von Guanidiumchlorid unterscheidet; und
(i) Wiederholen der gleichen Schritte (b) bis (h) außer in Schritt (b) mit einer neuen Probenlösung, die eine neue Probe umfasst, wodurch der Analyt in jeder der mehreren Flüssigkeitsproben detektiert wird.

13. Verfahren nach Anspruch 12, wobei der erste Immunokomplex aus Schritt (b) gebildet wird durch:
(b1) Mischen einer Probenlösung mit einer dualen Antikörperlösung und einer Reagenzlösung, um eine Mischung zu bilden, wobei die Probenlösung den Analyten umfasst, die duale Antikörperlösung den Anti-Hapten-Antikörper umfasst, der kovalent mit dem Einfang-Antikörper verknüpft ist, und die Reagenzlösung den Signal-Antikörper umfasst, der mit Fluoreszenzmarkierungen konjugiert ist; und
(b2) Tauchen der Sondenspitze in die Mischung, um den ersten Immunokomplex auf der Sondenspitze zu bilden.

14. Verfahren nach Anspruch 12, wobei der Immunokomplex aus Schritt (b) gebildet wird durch:
(b1) Tauchen der Sondenspitze in die duale Antikörperlösung, die den Anti-Hapten-Antikörper umfasst, der kovalent mit dem Einfang-Antikörper verknüpft ist;
(b2) Tauchen der Sondenspitze in eine Probenlösung, welche die Probe umfasst;
(b3) Tauchen der Sondenspitze in eine Reagenzlösung, die den Signal-Antikörper umfasst, der mit Fluoreszenzmarkierungen konjugiert ist, um den ersten Immunokomplex auf der Sondenspitze zu bilden.

15. Verfahren nach Anspruch 1, 6 oder 12, wobei der Einfang-Antikörper kovalent über Copolymere von Sucrose und Epichlorhydrin mit dem Anti-Hapten-Antikörper verknüpft ist.

## Revendications

1. Procédé de détection d'un analyte dans plusieurs échantillons liquides comprenant un analyte, comprenant les étapes suivantes :
(a) l'immersion d'une sonde dans une solution aqueuse ayant un pH de 6,0 à 8,5 pour prélire le signal fluorescent de l'extrémité de la sonde, la sonde comprenant un haptène immobilisé sur l'extrémité de la sonde, et le diamètre de la surface de l'extrémité étant ≤ 5 mm ;
(b) la formation d'un immunocomplexe comprenant l'analyte d'un échantillon, un anticorps de capture et un anticorps de signalisation sur l'extrémité de la sonde, l'anticorps de capture et l'anticorps de signalisation étant deux anticorps différents contre l'analyte, l'anticorps de capture étant lié par covalence à un anticorps anti-haptène contre l'haptène et l'anticorps de signalisation étant conjugué à des marqueurs fluorescents ;
(c) l'immersion de l'extrémité de la sonde dans un récipient de lavage contenant une solution de lavage ;
(d) la détermination de la concentration de l'analyte dans l'échantillon en mesurant le signal fluorescent de l'immunocomplexe à l'extrémité de la sonde, en soustrayant le signal fluorescent prélu de (a), et en quantifiant le signal soustrait par rapport à une courbe d'étalonnage ;
(e) l'immersion de l'extrémité de la sonde dans une solution acide ayant un pH d'environ 1,0 à 4,0 pour éluer l'immunocomplexe de l'extrémité de la sonde ; et
(f) la répétition des étapes (a) à (e), à l'exception de l'étape (b), avec l'analyte d'un nouvel échantillon, ce qui permet de détecter l'analyte dans chacun des plusieurs échantillons liquides.

2. Procédé selon la revendication 1, dans lequel l'immunocomplexe de l'étape (b) est formé par :
(b1) le mélange d'une solution d'échantillon avec une solution d'anticorps double et une solution de réactif pour former un mélange, la solution d'échantillon comprenant l'analyte, la solution d'anticorps double comprenant l'anticorps anti-haptène lié par covalence à l'anticorps de capture, et la solution de réactif comprenant l'anticorps de signalisation conjugué à des marqueurs fluorescents ; et
(b2) l'immersion de l'extrémité de la sonde dans le mélange pour former l'immunocomplexe sur l'extrémité de la sonde.

3. Procédé selon la revendication 1, dans lequel l'immunocomplexe de l'étape (b) est formé par :
(b1) l'immersion de l'extrémité de la sonde dans une solution d'anticorps double comprenant l'anticorps anti-haptène lié par covalence à l'anticorps de capture ;
(b2) l'immersion de l'extrémité de la sonde dans une solution d'échantillon comprenant l'échantillon ;
(b3) l'immersion de l'extrémité de la sonde dans une solution de réactif comprenant l'anticorps de signalisation conjugué à des marqueurs fluorescents pour former l'immunocomplexe sur l'extrémité de la sonde.

4. Procédé selon la revendication 1, 2 ou 3, dans lequel :
i) la solution acide de l'étape (e) a un pH de 1,5 à 2,5
ii) l'extrémité de la sonde est exposée à la solution acide une fois pendant 10 secondes à 2 minutes, ou
iii) l'extrémité de la sonde est exposée à un traitement par impulsions de 2 à 5 cycles du traitement par solution acide suivi d'une neutralisation dans le récipient de lecture pendant 10 à 20 secondes.

5. Procédé selon la revendication 1, 2 ou 3, dans lequel l'haptène est la fluorescéine, la digoxiginine ou la biotine.

6. Procédé de détection d'un analyte dans plusieurs échantillons liquides, comprenant les étapes suivantes :
(a) l'immersion d'une sonde dans une solution aqueuse ayant un pH de 6,0 à 8,5 pour prélire le signal fluorescent de l'extrémité de la sonde, la sonde comprenant un premier haptène immobilisé sur l'extrémité de la sonde, et le diamètre de la surface de l'extrémité étant ≤ 5 mm ;
(b) la formation d'un premier immunocomplexe comprenant l'analyte d'un échantillon, un anticorps de capture et un anticorps de signalisation sur l'extrémité de la sonde, l'anticorps de capture et l'anticorps de signalisation étant deux anticorps différents contre l'analyte, l'anticorps de capture étant lié par covalence à un anticorps anti-premier haptène contre le premier haptène et l'anticorps de signalisation étant conjugué à un deuxième haptène, le premier haptène et le deuxième haptène étant différents ;
(c) l'immersion de l'extrémité de la sonde dans une solution de lavage ;
(d) l'immersion de l'extrémité de la sonde dans une solution d'amplification comprenant un anticorps anti-deuxième haptène ou de la streptavidine conjuguée à des marqueurs fluorescents pour former un deuxième immunocomplexe comprenant l'analyte, l'anticorps de capture, l'anticorps de signalisation, le deuxième haptène et l'anticorps anti-deuxième haptène ou la streptavidine sur l'extrémité de la sonde ;
(e) la détermination de la concentration de l'analyte dans l'échantillon en mesurant le signal fluorescent du deuxième immunocomplexe à l'extrémité de la sonde, en soustrayant le signal fluorescent prélu de (a), et en quantifiant le signal soustrait par rapport à une courbe d'étalonnage ;
(f) l'immersion de l'extrémité de la sonde dans une solution acide ayant un pH d'environ 1,0 à 4,0 pour éluer les immunocomplexes de l'extrémité de la sonde ; et
(g) la répétition des mêmes étapes (a) à (f) à l'exception de l'étape (b) avec l'analyte d'un nouvel échantillon, ce qui permet de détecter l'analyte dans chacun des plusieurs échantillons liquides.

7. Procédé selon la revendication 6, dans lequel le premier immunocomplexe de l'étape (b) est formé par :
(b1) le mélange d'une solution d'échantillon avec une solution d'anticorps double et une solution de réactif pour former un mélange, la solution d'échantillon comprenant l'analyte, la solution d'anticorps double comprenant l'anticorps anti-premier haptène lié par covalence à l'anticorps de capture, et la solution de réactif comprenant l'anticorps de signalisation conjugué au deuxième haptène ; et
(b2) l'immersion de l'extrémité de la sonde dans le mélange pour former le premier immunocomplexe sur l'extrémité de la sonde.

8. Procédé selon la revendication 6, dans lequel le premier immunocomplexe de l'étape (b) est formé par :
(b1) l'immersion de l'extrémité de la sonde dans une solution d'anticorps double comprenant l'anticorps anti-premier haptène lié par covalence à l'anticorps de capture ;
(b2) l'immersion de l'extrémité de la sonde dans une solution d'échantillon comprenant l'échantillon ;
(b3) l'immersion de l'extrémité de la sonde dans une solution de réactif comprenant l'anticorps de signalisation conjugué au deuxième haptène pour former le premier immunocomplexe sur l'extrémité de la sonde.

9. Procédé selon la revendication 6, 7 ou 8, dans lequel la solution d'amplification comprend un anticorps anti-deuxième haptène conjugué aux marqueurs fluorescents.

10. Procédé selon la revendication 6, 7 ou 8, dans lequel le premier haptène et le deuxième haptène sont choisis dans le groupe constitué par : la fluorescéine, la digoxiginine et la biotine, de préférence dans lequel le premier haptène n'est pas la biotine, le deuxième haptène est la biotine, et la solution d'amplification comprend de la streptavidine conjuguée aux marqueurs fluorescents.

11. Procédé selon la revendication 6, 7 ou 8, dans lequel la solution d'amplification comprend des copolymères de saccharose et d'épichlorhydrine liés au conjugué de l'anticorps anti-deuxième haptène ou de la streptavidine et des marqueurs fluorescents.

12. Procédé de détection d'un analyte dans plusieurs échantillons liquides, comprenant les étapes suivantes :
(a) l'immersion d'une sonde dans une solution aqueuse ayant un pH de 6,0 à 8,5 pour prélire le signal fluorescent de l'extrémité de la sonde, la sonde comprenant un haptène immobilisé sur l'extrémité de la sonde, et le diamètre de la surface de l'extrémité étant ≤ 5 mm ;
(b) la formation d'un immunocomplexe comprenant l'analyte d'un échantillon, un anticorps de capture et un anticorps de signalisation sur l'extrémité de la sonde, l'anticorps de capture et l'anticorps de signalisation étant deux anticorps différents contre l'analyte, l'anticorps de capture étant lié par covalence à un anticorps anti-haptène contre l'haptène et l'anticorps de signalisation étant conjugué à des marqueurs fluorescents ;
(c) l'immersion de l'extrémité de la sonde dans un récipient de lavage contenant une solution de lavage ;
(d) la lecture d'un premier signal fluorescent de l'immunocomplexe à l'extrémité de la sonde ;
(e) l'immersion de l'extrémité de la sonde dans une solution acide ayant un pH d'environ 1,0 à 4,0 et contenant du chlorure de guanidium ;
(f) la lecture d'un deuxième signal fluorescent de l'extrémité de la sonde traitée à l'acide,
(g) la soustraction du deuxième signal fluorescent du premier signal fluorescent, et la quantification du signal soustrait par rapport à une courbe d'étalonnage ;
(h) l'immersion de l'extrémité de la sonde dans un agent chaotrope différent du chlorure de guanidium ; et
(i) la répétition des mêmes étapes (b) à (h), à l'exception de l'étape (b) avec une nouvelle solution d'échantillon comprenant un nouvel échantillon, ce qui permet de détecter l'analyte dans chacun des plusieurs échantillons liquides.

13. Procédé selon la revendication 12, dans lequel le premier immunocomplexe de l'étape (b) est formé par :
(b1) le mélange d'une solution d'échantillon avec une solution d'anticorps double et une solution de réactif pour former un mélange, la solution d'échantillon comprenant l'analyte, la solution d'anticorps double comprenant l'anticorps anti-haptène lié par covalence à l'anticorps de capture, et la solution de réactif comprenant l'anticorps de signalisation conjugué à des marqueurs fluorescents ; et
(b2) l'immersion de l'extrémité de la sonde dans le mélange pour former le premier immunocomplexe sur l'extrémité de la sonde.

14. Procédé selon la revendication 12, dans lequel l'immunocomplexe de l'étape (b) est formé par :
(b1) l'immersion de l'extrémité de la sonde dans une solution d'anticorps double comprenant l'anticorps anti-haptène lié par covalence à l'anticorps de capture ;
(b2) l'immersion de l'extrémité de la sonde dans une solution d'échantillon comprenant l'échantillon ;
(b3) l'immersion de l'extrémité de la sonde dans une solution de réactif comprenant l'anticorps de signalisation conjugué à des marqueurs fluorescents pour former le premier immunocomplexe sur l'extrémité de la sonde.

15. Procédé selon la revendication 1, 6 ou 12, dans lequel l'anticorps de capture est lié par covalence à l'anticorps anti-haptène par l'intermédiaire de copolymères de saccharose et d'épichlorhydrine.
